# EUROPEAN PATENT SPECIFICATION

(11) **EP 3 571 508 B1**
(45) Date of publication and mention of the grant of the patent: **19.04.2023**
(21) Application number: 18741940.3
(22) Date of filing: 18.01.2018
(51) Int. Cl.: G01N 33/574, A61K 49/00, C12Q 1/02, C07K 16/00, C07K 16/28, C40B 30/04

(54) **DETECTING CANCER STEM CELLS USING A GLYCAN BIOMARKER**
NACHWEIS VON KREBSSTAMMZELLEN UNTER VERWENDUNG EINES GLYCAN-BIOMARKERS
DÉTECTION DE CELLULES SOUCHES CANCÉREUSES À L'AIDE D'UN BIOMARQUEUR À BASE DE GLYCANE

(30) Priority: 18.01.2017 US 201762447654 P
(43) Date of publication of application: 27.11.2019
(73) Proprietor: SRI International, Menlo Park, CA 94025-3539 (US)
(72) Inventor: WANG, Denong, Palo Alto CA 94303 (US)
(74) Representative: D Young & Co LLP
(86) International application number: PCT/US2018/014260
(87) International publication number: WO 2018/136649

(56) References cited:
- WO-A1-2016/201240
- US-A1- 2008 118 432
- HUABEI GUO ET AL: "O -Linked N -Acetylglucosamine ( O -GlcNAc) Expression Levels Epigenetically Regulate Colon Cancer Tumorigenesis by Affecting the Cancer Stem Cell Compartment via Modulating Expression of Transcriptional Factor MYBL1", JOURNAL OF BIOLOGICAL CHEMISTRY, vol. 292, no. 10, 17 January 2017 (2017-01-17), pages 4123-4137, XP055740289, US ISSN: 0021-9258, DOI: 10.1074/jbc.M116.763201
- DENONG WANG ET AL: "Carbohydrate Microarrays Identify Blood Group Precursor Cryptic Epitopes as Potential Immunological Targets of Breast Cancer", JOURNAL OF IMMUNOLOGY RESEARCH, vol. 2015, 1 January 2015 (2015-01-01), pages 1-9, XP055740351, US ISSN: 2314-8861, DOI: 10.1155/2015/510810
- WANG DENONG ET AL: "Chapter 15: Glycan Markers as Potential Immunological Targets in Circulating Tumor Cells", RETINAL DEGENERATIVE DISEASES: ADVANCES IN EXPERIMENTAL MEDICINE AND BIOLOGY, SPRINGER, US, PAGE(S) 275 - 284 , 1 January 2017 (2017-01-01), XP009523492, ISBN: 978-3-319-55947-6 Retrieved from the Internet: URL:http://link.springer.com/10.1007/978-3 -319-55947-6_15 [retrieved on 2017-05-31]
- MARIA TOLOUDI ET AL: "Correlation between Cancer Stem Cells and Circulating Tumor Cells and Their Value", CASE REPORTS IN ONCOLOGY, vol. 4, no. 1, 29 January 2011 (2011-01-29), pages 44-54, XP055390490, DOI: 10.1159/000324403
- WANG ET AL.: 'Exploring Glycan Markers for Immunotyping and Precision-targeting of Breast Circulating Tumor Cells' ARCH MED RES vol. 46, no. 8, November 2015, pages 642 - 650, XP029394167

## Description

Various diseases have cells with antigens that can be targeted for treatment purposes and/or other purposes. Epitopes of the antigens associated with diseases, such as cancer, can be used as biomarkers for targeting diagnosis or treatment. In cancer, circulating tumor cells (CTCs) and cancer stem cells (CSCs) have been identified as a prognostic factor in the development of cancer and progression to metastases. CSCs, in particular, are a progenitor of metastases and relapse in cancer patient. CTCs are rare cancer cells in blood circulation that are shed from the primary tumor and play key roles in disseminating metastatic tumor cells to remote sites. Detection of CTCs has been explored as a non-invasive "liquid biopsy" for tumor diagnosis and prognosis. CSCs, which are sometimes referred to as circulating cancer stem cells (cCSCs), belong to a subpopulation of undifferentiated tumor cells with embryonic characteristics. With epithelial-to-mesenchymal transition traits, CSCs are capable of escaping the primary tumor and entering the bloodstream as a subset of CTCs with high metastatic potential. Developing targeted immunotherapy to eradicate metastatic tumor cells *in vivo* is beneficial for precision tumor medicine.

Guo et al., Journal of Biological Chemistry (2017), 292(10): 4123-4137 discloses the epigenetic regulation of colon tumor development by O-GlcNAc.

Wang et al., Journal of Immunology Research (2015), 2015: 1-9 discloses carbohydrate microarrays used to reveal a cryptic O-glycan epitope of blood group precursors that is selectively expressed and surface-exposed in certain breast tumor cells.

Wang et al., Retinal Degenerative Diseases: Advances in Experimental Medicine and Biology, Springer, US (2017), Chapter 15: 275-284 discloses an experimental approach for characterizing tumor biomarkers overexpressed by circulating tumor cells (CTCs.

Toloudi et al., Case Reports in Oncology (2011), 4(1): 44-54 discusses potential correlations between cancer stem cells and circulating tumor cells.

WO 2016/201240 A1 discloses methods of testing anti-STn antibodies for their ability to target cancer stem cells .

Wang et al., Arch Med Res (2015), 46(8): 642-650 discloses an experimental approach to enable rapid screening of CTCs for glycan marker identification and characterization.

US 2008/118432 A1 discloses methods of monitoring cancer stem cells in patients undergoing cancer therapy.

Identifying CTC-specific and CSC-specific cell-surface biomarkers is substantially challenging. First, CTCs and CSCs originate from self-epithelial cells. Biomarkers currently in use for detection and isolation of these cells, such as the cell surface marker epithelial cell adhesion molecule (EpCAM), are commonly expressed by normal epithelial cells. The lack of specific immunological targets to detect CTCs and CSCs is a road block to development of highly specific immunotherapy against tumor metastasis. Second, CTCs and CSCs are extremely rare. The number of CTCs detectable in blood is approximately 1 CTC per 10⁶-10⁷ of peripheral mononuclear blood cells (PBMCs). The number of detectable CSCs is even smaller than CTCs. Conventional molecular and cellular techniques may not detect them and the molecular targets they express.

The above issues as well as others have presented challenges to identifying and isolating human antibodies for a variety of applications.

### SUMMARY

The present disclosure is directed to overcoming the above-mentioned challenges and others related to detecting CSCs in blood circulation (cCSCs) or tissue-associated CSCs (tCSCs) as discussed above and in other implementations. The present disclosure is exemplified in a number of implementations and applications, some of which are summarized below as examples.

Various aspects of the present disclosure are directed to methods for detecting CSCs in a biological sample of a subject via a glycan biomarker.

In particular, the present invention provides a method for detecting circulating cancer stem cells (CSCs) in a biological sample of a subject, the method comprising:
causing a physical interaction between the biological sample and an antibody by exposing the biological sample to the antibody; and
analyzing a cell population of the biological sample by:
   determining a presence of CSCs within the cell population by detecting binding between the antibody and a glycan biomarker, the glycan biomarker including at least one chain selected from the group consisting of:
   polylactosamine chains, oligosaccharide chains, and combinations thereof, the at least one chain having branches selected from the group consisting of: IIβ (Galβ1,4GlcNAcβ1,6), Iβ(Galβ1,3GlcNAcβ1,6), IIβ/Iβ (Gal β1, 4/3GlcNAc β1,6) -moieties, and combinations thereof; and
   detecting a level of the CSCs within the cell population of the biological sample responsive to the detection of binding between the antibody and the glycan biomarker.

In various embodiments, analyzing the cell population further includes:
immunotyping the CSCs and circulating tumor cells (CTCs) within the cell population responsive to the detected binding between the antibody and the glycan biomarker.

In various embodiments, the method defined herein further includes:
classifying the cell population as CSCs, circulating tumor cells (CTCs), and benign cells responsive to the detected binding between the antibody and the glycan biomarker. The cells of the cell population may be classified as blood circulating CSCs (cCSCs), and tissue-associated CSCs (tCSCs), and CTCs in blood circulation (cCTC).

In various embodiments, the glycan biomarker includes an epitope of a blood group precursor antigen and the antibody is an anti-tumor monoclonal antibody is C1, HAE3, or G1.

In various embodiments, determining the presence of the CSCs in the biological sample further includes using optical circuitry to detect the binding by identifying the specific binding of the glycan biomarker by the antibody within the biological sample.

In various embodiments, the glycan biomarker includes an O-core cryptic epitope of a blood group precursor antigen, the blood group precursor antigen selected from the group consisting of: Tij II 20% fraction 2nd 10% (Tij II), OG 10% 2X (OG), and a combination thereof.

In various embodiments, analyzing the cell population of the biological sample further includes immunotyping the CSCs and circulating tumor cells (CTCs) within the biological sample responsive to the detected binding between the antibody and the glycan biomarker and based on differences in morphology of the CSCs and CTCs.

In various embodiments, causing the physical interaction between the biological sample and the antibody further includes:
immobilizing the biological sample on a substrate and exposing the immobilized biological sample to the antibody and a detection agent, wherein the presence of the glycan biomarker within the biological sample results in the antibody binding to the glycan biomarker and binding of the detection agent to an FC segment of the antibody.

In various embodiments, determining the presence of the CSCs within the cell population further includes:
identifying the presence of the antibody bound to the glycan biomarker within the cell population, and
detecting the presence of the CSCs within the cell population of the biological sample responsive to the identified presence of the antibody bound to the glycan biomarker; and
analyzing the cell population of the biological sample includes characterizing at least a portion of the cell population as CSCs responsive to the identified presence of the antibody bound the glycan biomarker and using morphological and immunological analysis via a fiber-optic array scanning technology (FAST) scan to distinguish CSCs from benign cells in the cell population.

In various embodiments, analyzing the cell population of the biological sample further includes classifying at least a portion of the cell population as CSCs and at least another portion as circulating tumor cells, by morphological and immunological analysis.

In various embodiments, the biomarker includes a plurality of oligosaccharide chains having branches selected from the group consisting of: IIβ (Galβ1,4GlcNAcβ1,6), Iβ(Galβ1,3GlcNAcβ1,6), and combinations thereof, the method further including detecting a presence of metastatic cancer in the biological sample responsive to the detected level of the CSCs within the cell population.

In various embodiments, analyzing the cell population further includes comparing the detected level of the CSCs within the cell population to a previously determined level of CSCs of a different biological sample of the subject.

In various embodiments, the glycan biomarker includes an O-core cryptic epitope of a blood group precursor antigen, the blood group precursor antigen being selected from the group consisting of: Tij II 20% fraction 2nd 10% (Tij II), OG 10% 2X (OG), and a combination thereof; and
analyzing the cell population further includes identifying cancerous cells associated with an epithelial cancer in response to the detected level of the CSCs.

In various embodiments, the method defined herein further includes determining an efficacy of a drug candidate compound for treatment of cancer in a subject by:
obtaining a plurality of biological samples from blood or tissue of a subject before and after treatment with the drug candidate compound, the biological samples comprising a cell population suspected of containing CSCs, the plurality of biological samples including the biological sample;
causing physical interactions between each of the plurality of biological samples and the antibody by exposing the plurality of biological samples to the antibody; and
analyzing the cell population by identifying levels of the CSCs within the biological samples before treatment with the drug candidate compound compared to after treatment with the drug candidate compound, wherein the presence of a decreased number of the CSCs after treatment compared to a number of the CSCs before treatment indicates a relative efficacy of the drug candidate compound.

The above overview is not intended to describe each illustrated embodiment or every implementation of the present disclosure. The figures and detailed description that follow more particularly exemplify these embodiments.

### BRIEF DESCRIPTION OF THE DRAWINGS

Various example embodiments may be more completely understood in consideration of the following detailed description in connection with the accompanying drawings, in which:
FIG. 1 illustrates an apparatus;
FIG. 2 illustrates an example process for detecting the presence of CSCs and specific immunotypes of CSCs in a biological sample, in accordance with various embodiments; the step of obtaining a blood sample from a patient as depicted in FIG. 2 is not encompassed by the present invention;
FIG. 3 illustrates an example process for assessing a cell population of a biological sample and/or assessing efficacy of a treatment using a glycan biomarker, in accordance with various embodiments; the steps of obtaining a blood sample from a patient, and revising and administering a revised treatment are not encompassed by the present invention;
FIGs. 4A-4C illustrate example substrates for detecting the presence of the glycan biomarker, in accordance with various embodiments;
FIG. 5A illustrates an example of a glycan-array, in accordance with various embodiments;
FIG. 5B illustrates an example of a resulting glycan biomarker, in accordance with various embodiments;
FIG. 5C illustrates an example of optical circuitry, in accordance with various embodiments;
FIG. 5D illustrates an example of a resulting immunotype of CTCs and CSCs from a scan of blood samples using optical circuitry, in accordance with various embodiments;
FIG. 6 illustrates an example of a blood group substance with a conserved O-glycan core and the epitopes recognized by antibody C1, HAE3, and G1, in accordance with various embodiments;
FIGs. 7A-7B illustrates example experimental results of a carbohydrate microarray used to identify glycan biomarkers using antibody HAE3, in accordance with various embodiments;
FIGs. 8A-8B illustrate example experimental results of expression of surface tumor biomarkers, in accordance with various embodiments; and
FIGs. 9A-9B illustrate example experimental results of characterizing blood samples from five Stage IV breast cancer patients, in accordance with various embodiments;
FIGs. 10A-10D illustrate example experimental results of use of carbohydrate microarrays for detection of glycan biomarkers using antibodies G1 and HAE3, in accordance with various embodiments; and
FIG. 11 illustrates example experimental results of staining cell lines using antibody G1 and CSC biomarkers, in accordance with various embodiments.

While various embodiments discussed herein are amenable to modifications and alternative forms, aspects thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the claims. In addition, the term "example" as used throughout this application is only by way of illustration, and not limitation.

### DETAILED DESCRIPTION

The present invention provides a method for detecting circulating cancer stem cells (CSCs) in a biological sample of a subject as defined in claim 1. A presence of CSCs is determined, more specifically, by detecting the presence of a surface-CSC glycan biomarker. The glycan biomarker may be an epitope of a blood group precursor which specifically recognizes (e.g., binds to) an anti-tumor (glycan) monoclonal antibody. In certain implementations, aspects of the present disclosure have been shown to be beneficial when used in the context of exposing the biological sample, such as a human blood sample or tissue sample (e.g., biopsy specimen), to an antibody and detecting binding between the antibody and the glycan biomarker using a detection agent. In embodiments, the cell population of the biological sample is analyzed by immunotyping the CSCs, classifying cells as benign, CSCs, and circulating tumor cells (CTCs) based on the identified antibodies bound to the glycan biomarker. For example, the cells can be classified as CSCs in blood circulation (cCSCs), tissue-associated CSCs (tCSCs), and CTCs in blood circulation (cCTC). The analysis can be used to identify cancerous cells within the biological sample, monitor progress of cancer in the subject, and/or determine an efficacy of treatment for the subject over time. While the present disclosure is not necessarily limited to such applications, various aspects of the disclosure may be appreciated through a discussion of various examples using this context.

Accordingly, in the following description various specific details are set forth to describe specific examples presented herein. It should be apparent to one skilled in the art, however, that one or more other examples and/or variations of these examples may be practiced without all the specific details given below. In other instances, well known features have not been described in detail so as not to obscure the description of the examples herein. For ease of illustration, the same reference numerals may be used in different diagrams to refer to the same elements or additional instances of the same element.

The claimed method for identifying CSCs present in a biological sample uses an antibody that is specific for, e.g., binds to, a glycan biomarker. Tumor biomarkers can be overexpressed by cCSCs and/or tCSCs, and can be used for targeted immunotherapy against tumor metastasis. Surprisingly, the tumor biomarkers are surface-glycan biomarkers of CSCs, as well as CTCs, and are used to detect CSCs and further may be used to immunotype the CSCs. CSCs, as previously described, have a variety of functional, morphological, and size differences, including different functional groups, as compared to CTCs, such as co-expression of CSC markers, such as CD44+CD24- phenotype. The glycan biomarker, more specifically, may be an epitope of a blood group precursor antigen. For example, the glycan biomarker may be an O-core cryptic epitope of a blood group precursor antigen, the blood group precursor antigen including Tij II 20% fraction 2nd 10% (Tij II) and/or OG 10% 2X (OG). The glycan biomarker can bind to an anti-tumor antibody, such as C1, HAE3, and/or G1. Presence of the glycan biomarker can indicate the presence of cancerous cells in the subject, such as metastatic cancer in the subject and/or early stage cancer. As used herein, a glycan biomarker is interchangeably referred to as "CSC/CTC glycan biomarker" and "cell-surface CSC/CTC biomarker".

As may be appreciated by one of ordinary skill, experimental antibodies are proteins that can be used by the immune system to detect, neutralize, and/or kill various target cells which may be harmful to the host organism, such as tumor cells and pathogens. The antibody can recognize and bind to a unique molecule of the target cell, called an antigen, via a binding region of the antibody. An antibody bound to the antigen can directly or indirectly (e.g., by triggering other parts of the immune system), detect, neutralize, and/or kill the target cell. For example, the antibody HAE3, C1, and/or G1 binds to the glycan biomarker, which is an epitope of an antigen associated with CSCs and CTCs, and the binding is used to detect for the presence of CSCs and further may be used to immunotype the CSCs and/or CTCs. For more general and specific information on the antibodies HAE3, C1, and G1, which are sometimes herein referred to as anti-human carcinoma antigen (HCA) antibodies, reference is made to US Patent No. 5,693,763, entitled "Antibodies to human carcinoma antigen", filed June 6, 1995; Rongshan Li, et. al, "Frequent Expression of Human Carcinoma-Associated Antigen, a Mucin-Type Glycoprotein, in Cells of Prostatic Carcinoma", Archives of Pathology & Laboratory Medicine: December 2004, Vol. 128, No. 12, pp. 1412-1417; and Jorge L. Yao, et. al, "Overexpression of Human Carcinoma-Associated Antigen in Urothelial Carcinoma of the Bladder", Archives of Pathology & Laboratory Medicine: July 2004, Vol. 128, No. 7, pp. 785-787. As provided by the above-noted references, antibody HAE3 is deposited at the American Tissue Type Culture Collection (ATCC), Rockville, MD under accession no. HB-9467. HAE3, from which C1 is prepared (e.g., is subclone of the parent murine hybridoma, HAE3, as described further below) and G1 are available from Egenix, Inc., located in Rochester, New York, now Bantam Pharmaceutical, LLC. G1 is additionally available from Creative BioMolecules, Inc., located in Hopkinton, Massachusetts, now Curis, Inc., located in Cambridge, Massachusetts. HAE3 is additionally available from Maine Biotechnology Services, located in Portland, Maine.

In specific experimental embodiments, the glycan biomarkers of CSCs are identified using a carbohydrate microarray. The carbohydrate microarray includes a panel of carbohydrate antigens that are scanned against anti-tumor monoclonal antibodies (mAbs) to identify potential glycan biomarker therefrom. Flow cytometry and fiber-optic array scanning technology (FAST) is then applied to determine if the identified antigens are tumor-specific cell-surface biomarkers, e.g., are CSC glycan biomarkers. The antibodies identified can also be validated for performance in CSC and/or CTC-detection and immunotyping analysis using cancer patient's blood samples.

The glycan biomarkers can be used for identification of cancerous cells and/or treatment of the organism, such as for cancer treatment. The method of the present invention comprises steps wherein CSCs in a biological sample of a subject are detected by exposing the biological sample to an antibody, e.g., the anti-tumor mAb, and analyzing a cell population of the biological sample by determining the presence of CSCs in the biological sample by detecting binding between the antibody and the glycan biomarker, the glycan biomarker including at least one chain selected from the group consisting of polylactosamine chains, oligosaccharide chains, and combinations thereof, the at least one chain having branches selected from the group consisting of: IIβ (Galβ1,4GlcNAcβ1,6), Iβ(Galβ1,3GlcNAcβ1,6), IIβ/Iβ (Gal β1, 4/3GlcNAc β1,6) -moieties, and combinations thereof. Exposing the biological sample to the antibody causes a physical interaction between the antibody and the biological sample. In the present invention, the level of the CSCs within the cell population of the biological sample responsive to the detection of binding between the antibody and the glycan biomarker is detected. Antibodies bound to the glycan biomarker may be detected using a detection agent, which binds to the antibody or another epitope of the antigen associated with the glycan biomarker.

In specific embodiments, the physical interaction includes immobilizing the biological sample to a substrate, such as a glass substrate, a bead, or a nano or micro-array, and exposing the immobilized biological sample to the antibody. In other embodiments, the antibody is immobilized to a substrate and exposed to the biological sample. The substrate can be scanned to identify binding between the antibody and the glycan biomarker using optical circuitry by applying a detection agent that is conjugated to or specific for the anti-glycan antibody (e.g., the antibody bound to the glycan biomarker). The detection agent can be applied to the antibody prior to exposing the biological sample to the antibody or can be applied after exposing the biological sample to the antibody. The detection agent may be man-made. In response to identifying binding, the presence of CSCs within a cell population of the biological sample is detected and the CSCs (as well as the CTCs) can be immunotyped.

The detection agent can include a label and/or a second antibody. For example, the antibody itself may be labeled with a detection agent, such as with a fluorescence, enzymatic, or radioactive label and/or a second antibody (e.g., an anti-antibody that binds to the antibody). The second antibody, which is labeled, can be exposed to the substrate after allowing for binding between the biological sample and the antibody to occur and washing away unbound antibody substance. In other embodiments, the antibody can be immobilized to the substrate and then the immobilized antibody is exposed to the biological sample. A second antibody, that binds to a different epitope of the antigen and is labeled, is subsequently exposed to substrate.

The detected CSCs within the biological sample and/or the cell population of the biological sample can be further analyzed in various more specific embodiments. The analysis can include characterizing at least a portion of the cell population as CSCs responsive to the detected binding and using morphological and immunological analysis via a FAST scan to distinguish CSCs from benign cells and/or to immunotype the CSCs in the cell population. For example, the cells in the cell population can be classified as benign cells, CSCs, and CTCs based on the identified bound antibodies, differences in morphology, and the absence of an antibody bound to a glycan biomarker at portions of the substrate. The CSCs can further be immunotyped to classify a type of cancer present in the biological sample, such as a cell line of breast cancer, and which can be used to refine treatment of the subject. Treatment of the subject is not encompassed by the present invention.

Alternatively and/or in addition, in various embodiments, the detection of CSCs can be used to identify metastatic cancer, assess efficacy of treatment of the user, and/or assess efficacy of a drug candidate compound. In some embodiments, the detection of the presence of CSCs can be used to detect a presence of metastatic cancer in the subject. In the present invention, the level (e.g., frequency) of CSCs present within the cell population of the biological sample responsive to the detection of binding between the antibody and the glycan biomarker is detected. Additionally, the immunotype of CSCs may be characterized. The level and/or immunotype of CSCs can provide an indication of the stage of cancer and can be monitored over time to assess the development of cancer in the subject and the efficacy of treatment. The treatment can be adjusted in response to the assessment - this is not encompassed by the present invention. For example, the efficacy of a drug candidate compound can be assessed by comparing CSC levels and immunotype in a biological sample of subject prior to treatment with the drug candidate compound to CSC levels and immunotype in another biological sample of the subject after treatment. Further, the glycan biomarkers can be used for targeted immunotherapy against tumor metastasis based on the immunotype of CSCs and/or CTCs.

Also described herein is an apparatus that is not encompassed by the present invention, which may be used to perform the various methodologies described herein. The apparatus can include (high speed) optical circuitry and processing circuitry. An example optical circuitry is a fiber optic scanner, which includes a fiber optic bundle array, a laser, and imaging circuitry (e.g., camera), such as FAST as further described herein. The FAST system scans a biological sample with the laser and collects a high resolution image of the sample using the fiber optic array. As previously described, the biological sample may be plated on a substrate (e.g., glass slide) and can be attached to a stage. The substrate may be treated with a fluorescently, enzymatically, or radioactively labeled antibody (e.g., an antibody with a detection agent applied) and may be scanned using the optical circuitry. The optical circuitry can scan the entire biological sample and generate a digital image of the locations of an antibody bound to a glycan biomarker (via label). The optical circuitry and/or processing circuitry can identify cells (e.g., locations on the substrate) that do not result in binding of the antibody, and tissue and/or cellular compartment locations of the bound antibody. The processing circuitry can, responsive to the identification, identify CSCs, including cCSCs and tCSCs and, optionally, other CTCs. The classification between CSCs and CTCs can be made based on size, shape, and/or co-expression of CSC markers, such as CD44+CD24- phenotype. Further, the CSCs and/or CTCs can be immunotyped based on the detected binding of the antibody. In other cases, the antibody can be plated on the substrate and treated with the biological sample (and subsequently exposed to another antibody that is labeled prior to the scan). Although this disclosure describes scanning with the FAST system, one skilled in the art will recognize that other types of scanning can also serve the same purpose including those based on multispectral and/or hyperspectral imaging.

The apparatus can additionally include various circuitry such as processing circuitry for controlling the various instruments, memory circuitry for storing data sets, and various computer-readable instructions for controlling the optical circuitry and processing circuitry for analyzing data obtained therefrom. Optionally, the apparatus can include a microengraving platform. The microengraving platform includes a multiple-well array, an immuno-assay, and/or an immuno-sandwich.

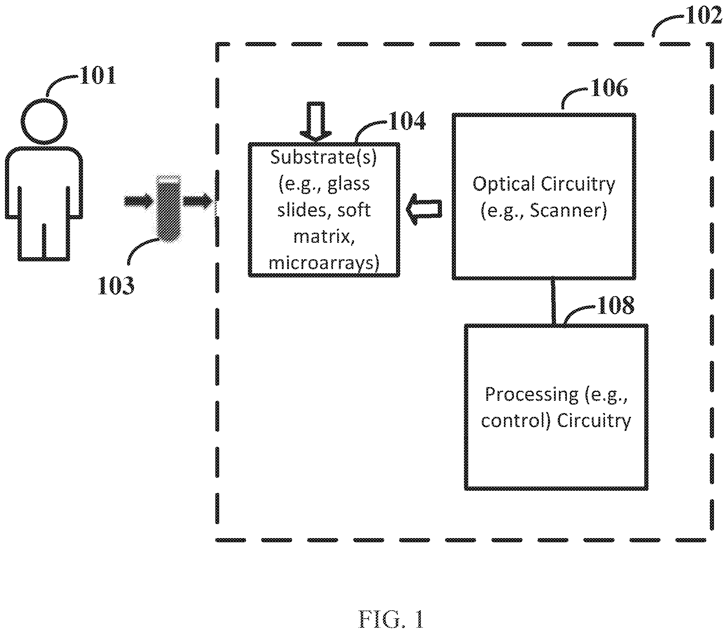

Turning now to the figures, FIG. 1 illustrates an example apparatus as described herein, which apparatus is not encompassed by the present invention. The apparatus 102 can scan a biological sample to detect the presence of CSCs using an antibody.

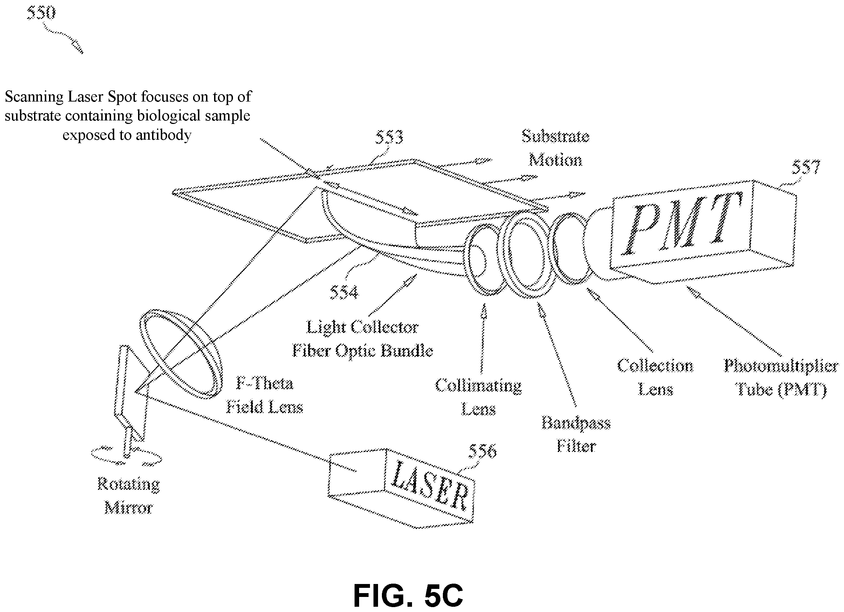

The apparatus 102 includes optical circuitry 106 combined with processing circuitry 108. The optical circuitry 106 can include a platform termed FAST, as further illustrated and described in connection with FIG. 5C. The optical circuitry 106 can be used to directly identify antibodies bound to glycan biomarkers from a biological sample immobilized on a substrate 104 (such as the blood sample 103 from a human 101 illustrated by FIG. 1). An example scanning technology, the FAST system is based on the concept of "Xeroxing" a blood sample with a scanning laser and collecting a high resolution capture image of the sample using a densely packed fiber optic array bundle. The FAST system can allow for rapid scanning of cells at speeds of between 1 million and 25 million cells per minute. For example, the optical circuitry 106 can scan the substrate 104 containing or otherwise associated with a biological sample of a subject. The subject may be suspected of having (or known to have) cancer. The biological sample can include a cell population that is exposed to an antibody, either a labeled antibody exposed to the biological sample or labeled via a secondary antibody (e.g., a labeled anti-antibody) via formation of an immuno-sandwich. In other related cases, the antibody can be immobilized to the substrate 104 and exposed to the biological sample. After washing away unbound cells from the biological sample, the substrate 104 can be exposed to a secondary antibody that is labeled and that binds to another epitope of an antigen associated with the glycan biomarker (e.g., another epitope of the blood group precursor antigen).

In specific embodiments, the exposure of the biological sample to the antibody, when the biomarker is present within the biological sample, causes (specific) binding between the antibody and the glycan biomarker. The antibodies bound to the glycan biomarker are labeled, in various embodiments, by applying a detection agent. The detection agent may be man-made and can include a fluorescent, enzymatic and/or radioactive label that is configured to bind to the antibody and/or a second antibody configured to bind to either the antibody or another epitope associated with the glycan biomarker. The second antibody may be labeled with a fluorescent, enzymatic and/or radioactive label. The detection agent can bind to antibody or the glycan biomarker via an epitope of the antibody or an epitope associated with the glycan biomarker. In some embodiments, the detection agent is applied to the antibody prior to exposing the biological sample to the antibody. In other embodiments, the detection agent is applied to the substrate 104 after exposing the biological sample to the antibody, and may bind to either the antibody or the epitope associated with the glycan biomarker (e.g., to the antigen associated with the glycan biomarker and via another epitope than the glycan biomarker).

The optical circuitry 106 scans for antibodies bound to the glycan biomarker that are bound to the substrate 104 responsive to the exposure to the antibody (or biological sample) and provides an image indicative of locations of the bound antibodies to the processing circuitry 108. For example, using the optical circuitry 106, glycan biomarkers bound to the antibody are identified via the detection agent and used to identify respective CSCs. The optical circuitry 106 and processing circuitry 108 can, responsive to the identified glycan biomarker bound to an antibody, provide an indication of the detected presence of CSCs. The presence of CSCs can indicate a presence of metastatic cancer in the subject and/or early stage cancer.

In some embodiments, the optical circuitry 106 and processing circuitry 108 can further assess a cell population of the biological sample (e.g., the blood sample 103 or a tissue sample, such as a biopsy specimen). The assessment can include identification and characterization of the cell population, such as using morphological and immunological analysis to characterize at least a portion of the cell population as CSCs, CTCs or benign. More specifically, identified glycan biomarkers bound to the antibodies can be used to distinguish CSCs and CTCs from benign cells. CSCs and CTCs can be distinguished from one another by identifying size, immunotype and morphology differences between the cells bound to the antibody via an optical scan of the substrate 104 by the optical circuitry 106. In more specific embodiments, once the cancerous (CSC or CTC tumor) cells are identified, further investigation can examine the characteristics of single cells by immunohistochemistry and other analyses such as fluorescence in situ hybridization (FISH), polymerase chain reaction (PCR), and single nucleotide polymorphism (SNP) analysis.

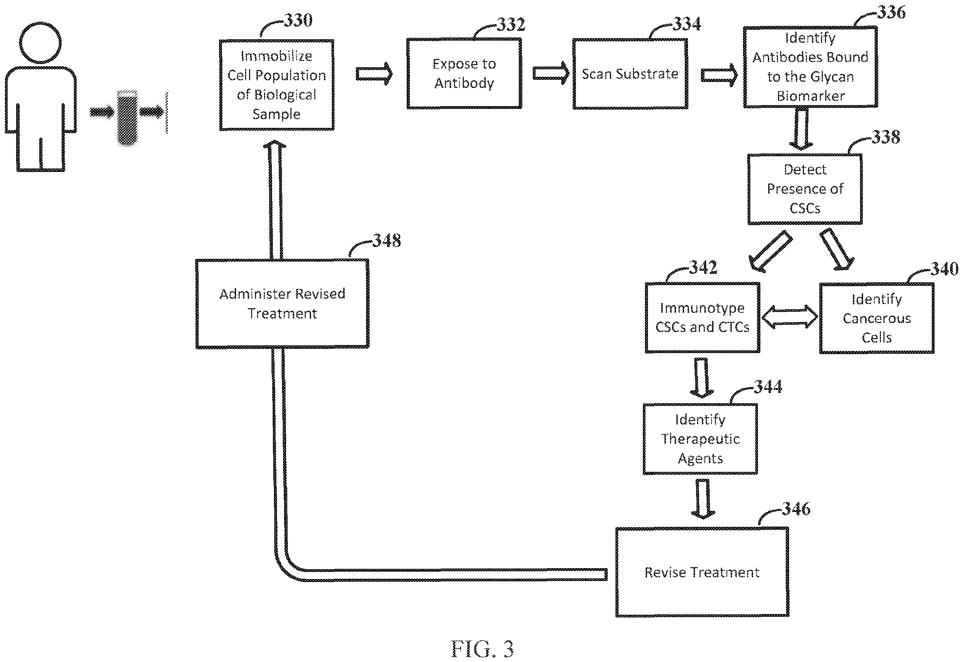

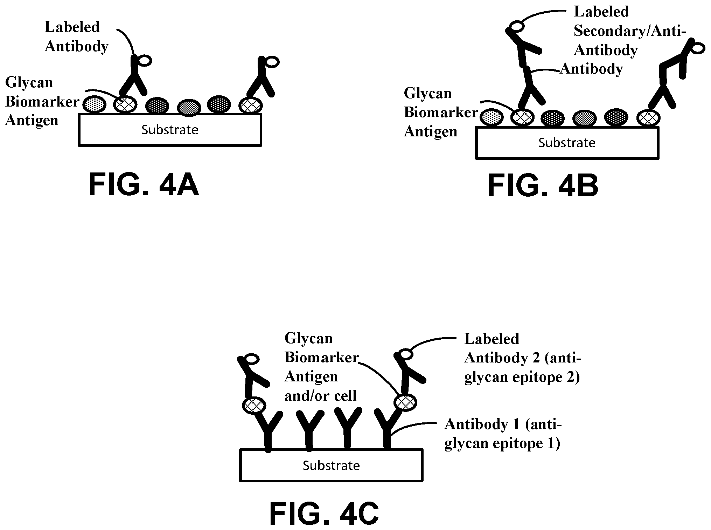

In some specific embodiments, the detection of CSCs is used to monitor treatment of the subject, as further illustrated by FIGs. 3-4. Additionally and/or alternatively, the detection of CSCs can be used assess the efficacy of a drug candidate compound, as illustrated by FIG. 4 and further discussed herein.

In various specific embodiments, exposing the biological sample to the antibody can include forming an immuno-assay. For example, a glass substrate may be coated with a biological sample suspected of containing the glycan biomarker (e.g., the epitope of the antigen) and used to form an immuno-sandwich by exposing the immobilized biological sample to the antibody (and optionally, a labeled anti-antibody). The immuno-sandwich is used to detect antibodies bound to the glycan biomarkers. The glass substrate can be treated with a detection agent that includes a labeled anti-antibody, and the optical circuitry 106 can scan the glass substrate to identify a signal (e.g., fluorescence) indicative of the labeled anti-antibody. If a CSC is present, the antibody binds to the glycan biomarker of the CSC on the glass substrate and the anti-antibody binds to the antibody. For example, the detection agent can bind to the FC segment of the antibody. Subsequently detected label (associated with the labeled anti-human detection antibody) indicates presence of the glycan biomarker. The anti-antibody can include various organism-specific antibodies, such as an anti-human antibodies, anti-horse antibodies, anti-dog antibodies, anti-cat antibodies, anti-fish antibodies, anti-cattle antibodies, anti-bird antibodies, among other organisms that have white blood cells which produce antibodies. As may be appreciated by one of ordinary skill in the art, the detection antibody used can be specific to the organism, such as an anti-horse detection antibody or an anti-dog detection antibody. Similarly, embodiments are not limited to first immobilizing the biological sample and can include immobilizing the antibody, as further illustrated by FIGs. 4A-4C.

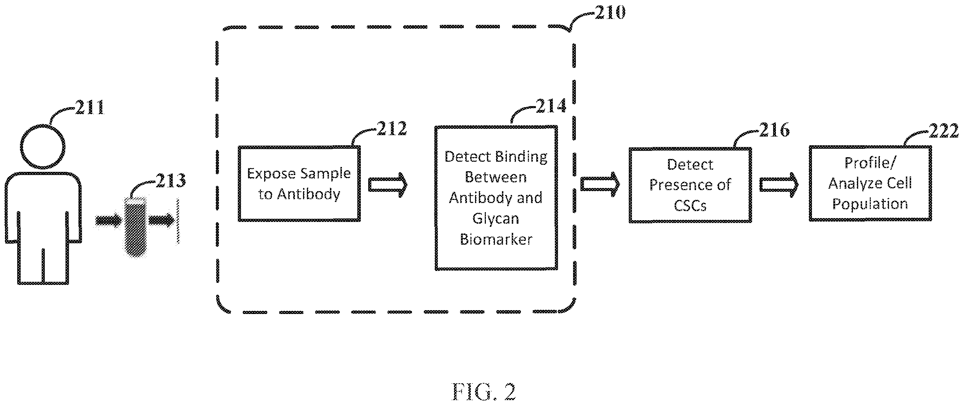

FIG. 2 illustrates an example process for detecting the presence of CSCs and specific immunotypes of CSCs in a biological sample, in accordance with various embodiments. More specifically, FIG. 2 illustrates an example method for detecting the presence of CSCs from a biological sample of a subject.

As illustrated by FIG. 2, a blood sample 213 may be obtained from a human 211 (not encompassed by the present invention). Although the figure illustrates the blood sample 213 being obtained directly from a human 211, the blood sample may be previously obtained and/or may be from other organisms and used to identify antibodies used to treat the particular organism (e.g., other vertebrates, such as horses, dogs, cats, cattle, fish, birds).

The blood cells may be immobilized, such as on a substrate, and attached to an apparatus 210. The apparatus 210 can include the apparatus 102, as previously illustrated and described by FIG. 1. In specific embodiments, the cell population for a blood sample is either fixed to one or more glass slide plates, or immobilized in a soft matrix such as agar or matrigel to maintain cell viability.

At 212, the immobilized blood cells are exposed to an antibody. For example, the immobilized blood cells can be exposed to an anti-tumor mAb, such as C1, HAE, and/or G1. The exposure, in specific examples, includes treating the substrate with a (fluorescently) labeled antibody. Blood cells that exhibit the glycan biomarker can bind to the labeled antibody. Further, as previously described, a detection agent can be applied, which can occur prior to or after immobilizing the blood cells. The detection agent can bind to the antibody bound to the glycan biomarker via an epitope of the antibody or another epitope associated with the glycan biomarker, as previously discussed.

At 214, the binding between the glycan biomarker of CSCs and/or CTCs and the antibody can be identified by scanning the substrate. The scan, in specific embodiments, can be by the optical circuitry, such as a FAST scan of the substrate, which identifies and locates the glycan biomarker-bound antibodies via the detection agent.

At 216, responsive to the detected binding, the presence of CSCs is determined. The presence of CSCs can be used to identify cancerous cells, diagnose the user with cancer, and/or further identify the stage of cancer. In specific embodiments, the cell population, at 222, is further analyzed and/or profiled. For example, the CSCs and/or CTCs can be immunotyped, in specific embodiments and as further described herein. Alternatively and/or in addition, the assessment can include classifying the cell population and determining levels (e.g., percentage of total cell population) of CSCs, CTCs, and/or benign cells within the cell population. The levels can be compared to thresholds and/or previously determined cell levels to assess the user progress, stage of cancer, efficacy of treatment, and/or to adjust a treatment (treatment not being within the scope of the invention). The presence of a decreased number of CSCs compared to a number of CSCs during a previous assessment can indicate successful treatment and/or regressing cancer in the subject. Similarly, the presence of an increased number of CSCs compared to the number of CSCs during the previous assessment can indicate treatment is not effective and/or progressing cancer in the subject.

FIG. 3 illustrates an example process for assessing a cell population of a biological sample and/or assessing efficacy of a treatment using a glycan biomarker, in accordance with various embodiments. As previously described, an apparatus, such as the apparatus 102 illustrated by FIG. 1, can be used to detect for the presence of CSCs in a biological sample of a user. The biological sample, in specific embodiments, is a blood sample of a subject suspected of having, or known to have, cancer. The blood sample comprises a cell population.

A blood sample is obtained from an organism, such as a human as illustrated, although this step is not encompassed by the present invention. At 330, the blood sample is immobilized on a substrate. In some specific embodiments, the cell population is deposited into a nanowell array. The nanowell array includes a plurality of wells arranged in an array, as further illustrated herein. Each well of the nanowell array can have an individual blood cell deposited therein. Further, the wells can include a cell culture media that allows for the cells deposited in the wells to remain viable.

At 332, a physical interaction between the blood sample and an antibody is caused by exposing the blood sample to the antibody. In some embodiments, the exposure includes exposing the substrate (e.g., glass substrate or nanowell array) to a solution containing the antibody that is labeled. The antibody may be labeled with a detection agent, such as a fluorescent, enzymatic, or radioactive label and/or second antibody. The detection agent can be used for identifying the CSCs and for subsequently phenotyping the blood cells. In other specific embodiments, the substrate is further exposed, after washing away unbound antibodies or cells, to a detection agent, such as a secondary antibody (e.g., an anti-antibody or a second antibody that binds to another epitope of the antigen associated with the glycan biomarker) that is labeled. Although embodiments are not so limited, and can include exposing the substrate, which has immobilized antibodies thereon, to the biological sample.

At 334, the substrate is scanned to identify binding between the glycan biomarker and the antibody using optical circuitry. The scan by the optical circuitry can be used to identify the locations on the glass slide or other substrates that reveal discrete spots associated with the detection agent and that correspond to an antibody bound to the glycan biomarker, at 336. As a specific example, if the blood cell exhibits the glycan biomarker, the antibody binds to the glycan biomarker on the glass slide. A detection agent, such as an anti-human antibody, can be applied to the substrate. For example, the anti-human IgG antibody, which is fluorescently, enzymatically, or radioactively labeled and washed over the glass slide, binds to the antibody and results in a signal, such as fluorescent emission, when scanned by the optical circuitry.

In response to identifying antibodies bound to the glycan biomarker, at 338, the presence of CSCs can be detected. In some embodiments, detecting the presence can further include classifying the cell population. For example, CSCs can be distinguished from CTCs among the cell population based on size, morphology, and cryptological differences. In other embodiments, CSCs can be distinguished from benign cells responsive to the detected bound antibodies. Optionally, at 340, in response to the detected presences of CSCs (or in response to the identified antibodies bound to the glycan biomarker), the subject can be identified as having cancerous cells, and optionally, diagnosed with cancer. More specifically, the subject can be analyzed for having metastatic cancer.

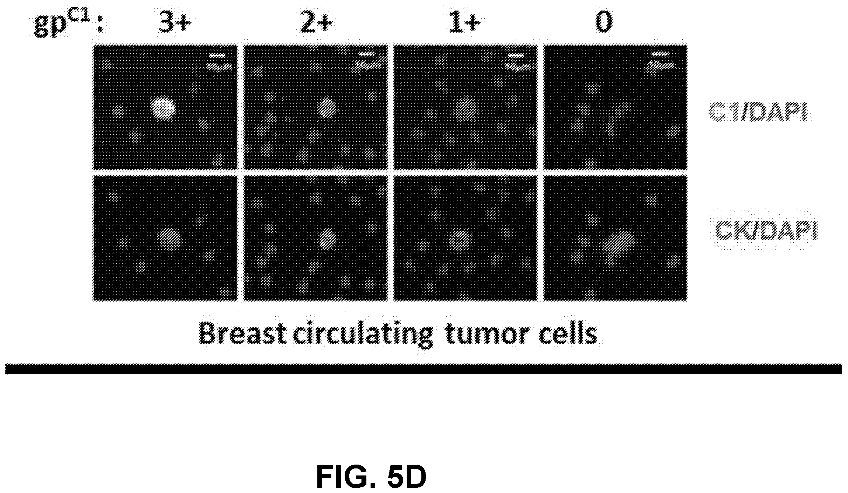

In some specific embodiments, based on the assessment of the cell population, at 342, the CTCs and/or CSCs can be immunotyped. Immunotyping the CSCs and/or CTCs may be used to further refine the assessment of cancerous cells (and optionally, the diagnosis), at 340, such as indicating a stage or cell line of cancer. In various embodiments, different cancer cell lines express different levels of the glycan biomarker. As an example and as further illustrated herein, two triple negative cell lines (BT-549 and MDA-MB-468) and two ER+PR+ cell lines (T-47D and MCF-7) are strongly HAE3 positive and Sk-BR-3 is intermediately HAE3 positive. Immunotyping the CSCs (and/or CTCs) can be based on the detected binding of the antibody including a percentage of cells that bind to the antibody (e.g., percentage of staining from the label of the detection agent) and an intensity of the signal associated with the binding (e.g., intensity of the staining from the label of the detection agent). More specifically, the immunotyping can be based on the percentage or level of CTCs and/or CSCs captured that express levels of the glycan biomarker above a threshold intensity (e.g., based on numeric values of 0, 1+, 2+, 3+ as illustrated by FIG. 5D). Biological samples having a percentage of cells that are bound to the antibody, e.g., percentage of stained cells, above a first threshold (e.g., 5%, 40%, 50%) and that have a signal intensity above a second threshold (e.g., 2+ or more, 1+_or more) can be used to immunotype the CSCs and/or CTCs. As a specific example, a biological sample exhibiting triple negative cells lines can be associated with a percentage of stained cells of 50% or more with a signal intensity of 2+ or more, although embodiments are not so limited. For example, various different quantifications of the expression signal intensities can be used and embodiments are not limited to the numerical values of 0, 1+, 2+, and 3+.

Based on the immunotyping, at 344, candidate therapeutic agents can be identified. The candidate therapeutic agents can include antibodies identified as useful in therapy for the specific immunotype of CTCs and/or CSCs, Car-T cells, and immunotype-cytokine, among other types of specific therapy and/or agents. The term "therapeutic agent" is used herein interchangeably with the term "drug candidate". Optionally, at 346, the identified therapeutic agent(s) can be used to refine the treatment for the subject. Treatment is not encompassed by the present invention.

In the present invention, the level of the CSCs within the cell population of the biological sample responsive to the detection of binding between the antibody and the glycan biomarker is detected. The level of CSCs may be used to further refine the assessment of the metastatic potential of the cancerous cells (and optionally, the diagnosis although this is not encompassed by the present invention). Additionally and/or alternatively, the levels of CSCs can be used to assess the efficacy of treatment for the user, to revise treatment for the user (not encompassed by the present invention), and/or assess efficacy of a drug candidate. For example, the levels of CSCs can be compared to a previous level of CSCs and/or immunotype of CSCs which may have been determined prior to a particular treatment or earlier in time. Based on the comparison, an efficacy of treatment for the user (or of a drug candidate compound in general) can be provided. More specifically, if the CSC levels decreased after treatment, the treatment may be considered effective. By contrast, greater levels of CSCs after treatment may indicate an ineffective treatment. Optionally, based on the efficacy assessment, the treatment for the user can be revised (treatment is not encompassed by the present invention). Example revisions can include adjusted dosages of drug compound(s), different drug compound(s), and additional treatments, among other revisions.

As a specific example, assessment of CSC levels and/or immunotype can be used to determine an efficacy of a drug candidate compound for treatment of cancer. The subject (and optionally many subjects) suspected of having cancer can be administered an amount of a drug candidate compound (not encompassed by the present invention). Biological samples are obtained from blood of the subject before and after treatment with the drug candidate compound, the biological samples comprising a cell population suspected of having CSCs. A physical interaction is caused between the biological samples and an antibody by exposing the biological samples to the antibody. A presence of CSCs in the biological sample can be determined by identifying the antibodies bound to the glycan biomarker within the cell population and which are bound to a detection agent, and detecting a presence or absence of the glycan biomarker in the biological samples responsive to the identified antibody bound to the glycan biomarker. The cell population can be analyzed to identify levels of CSCs and/or the immunotype of CSCs in the biological samples before treatment with the drug candidate compound compared to after treatment with the drug candidate compound. This process can be repeated for the same subject, many subjects, different candidate drugs, and/or different amounts of a specific drug candidate compound. The presence of a decreased number of CSCs after treatment compared to a number of CSCs before treatment can indicate a relative efficacy of the drug candidate compound in treating the cancer in the subject. In response to a decreased level of CSCs after treatment (and perhaps assessment of other side effects of the drug candidate compound), the patient may be given an increased dosage of the drug candidate, although this is not encompassed by the present invention.

Although FIGs. 2-3 illustrate a biological sample including a blood sample, embodiment are not so limited. For example, the biological sample can include a tissue sample, such as a biopsy specimen from tissue of the subject.

In various embodiments, the biological sample can be exposed to the antibody in a variety of ways. For example, the biological sample can be immobilized on a substrate or the antibody can be immobilized on the substrate. The substrate can include a bead, a glass slide and/or a matrix (e.g., agar or matrigel). The immobilized biological sample or the antibody can then be exposed to the antibody or the biological sample, respectively. The antibody can be labeled with a detection agent, such as with a fluorescent label, or the antibody as bound to the glycan biomarker can be exposed to a second antibody that is labeled, such as a secondary antibody configured to bind to a different epitope of the antigen associated with the glycan biomarker or an anti-antibody configured to bind to an epitope of the antibody. In some embodiments, the substrate coated with the antibodies bound to the glycan biomarker is treated with a labeled anti-human (or other anti-organism) antibody. The substrate can be washed and tagged with fluorescent anti-human IgG antibody. The anti-human IgG antibody can bind to antibodies present on the substrate and which are bound to the glycan biomarker of the antigen that is coated on the substrate.

FIGs. 4A-4C illustrate example substrates for detecting the presence of the glycan biomarker for CSCs, in accordance with various embodiments. As illustrated, in some embodiments, the exposure of the biological sample to the antibody can be used to form an immuno-assay, such as an immuno-sandwich.

In some embodiments, as illustrated by FIGs. 4A and 4B, the biological sample can first be immobilized to a substrate. The immobilized biological sample is then exposed to the antibody. As illustrated by FIG. 4A, the antibody can be directly labeled with a detection agent. The substrate can be washed to remove unbound antibody substance and scanned to detect antibodies bound to the glycan biomarker (e.g., a glycan biomarker antigen that is expressed on the CSC and/or CTC cell surface). In other embodiments, the substrate is washed to remove unbound antibody substance and then further exposed to a detection agent, such as a labeled secondary/anti-antibody that binds to the antibody, as illustrated by FIG. 4B. The substrate is then washed (to remove unbound detection agent) and scanned to detect the antibody bound to the glycan biomarker.

In other embodiments, as illustrated by FIG. 4C, the antibody can be immobilized to the substrate. The antibody can be a first antibody that binds to a first epitope of the glycan biomarker (e.g., anti-glycan epitope 1), such as the antibodies C1, HAE3, and/or G1. The immobilized antibody can then be exposed to the biological sample. The substrate can then be washed to remove unbound biological sample (which can be analyzed before or after to determine a total cell population in the biological sample and to assess the full population). A detection agent can be applied to the substrate, such as a labeled secondary antibody, e.g., the illustrated labeled antibody 2, that is specific to a different epitope of the antigen associated with the glycan biomarker (e.g., anti-glycan epitope 2). After washing the substrate to remove unbound secondary antibody substances, the substrate can be scanned to detect antibodies, e.g., antibody 1, bound to the glycan biomarker via the labeled antibody 2.

Although the embodiments of FIGs. 4A-4C illustrate a flat substrate, such as a glass substrate, embodiments are not so limited and can include a variety of different substrates, such as beads and arrays.

Embodiments in accordance with the present disclosure include detecting the presence of a glycan biomarker. Somewhat suprisingly, given the differences in size, morphology, and function, the glycan biomarker can be used to identify and/or immunotype CSCs and CTCs. The glycan biomarker can be an epitope of a blood group precursor antigen. More specifically, the glycan biomarker can bean O-core cryptic epitope of a blood group precursor antigen selected from the group consisting of: Tij II 20% fraction 2nd 10% (Tij II), OG 10% 2X (OG), and a combination thereof. The glycan biomarker includes at least one chain selected from the group consisting of polylactosamine chains, oligosaccharide chains, and combinations thereof. The at least one chain has branches selected from the group consisting of: IIβ (Galβ1,4GlcNAcβ1,6), Iβ(Galβ1,3GlcNAcβ1,6), IIβ/Iβ (Gal β1, 4/3GlcNAc β1,6) -moieties, and combinations thereof. More specifically, the glycan biomarker can include a plurality of chains selected from the group consisting of polylactosamine chains, oligosaccharide chains, and combinations thereof. Each of the plurality of chains can have branches selected from the group consisting of IIβ (Galβ1,4GlcNAcβ1,6), Iβ(Galβ1,3GlcNAcβ1,6), IIβ/Iβ (Gal β1, 4/3GlcNAc β1,6) -moieties, and combinations thereof. The glycan biomarker can bind to an anti-tumor mAb, such as C1, G1 orHAE3.

### More Specific/Experimental Embodiments

In specific experimental embodiments, a glycan biomarker is used to detect the presence of CSCs and CTCs, which is expressed by various cancer tumor cells including ovarian and breast tumor cells. Using the above-described techniques, glycan biomarkers are detected using mAbs, such as C1 and HAE3. In specific experiment embodiments, a variety of glycomic tools are used to uncover glycan biomarkers of CTCs and CSCs. Glycomics tools are used to probe cell-surface glycan biomarkers of breast CTCs (bCTCs). Specifically, carbohydrate microarrays are applied to screen anti-tumor antibodies to identify those that are specific for tumor glycan biomarkers. A high-speed FAST scan is then applied to verify whether the identified targets are CTC-specific cell-surface biomarkers.

Exploring glycan biomarkers of bCTCs and breast cancer stem cells (bCSCs) is useful in tumor biomarker discovery. Although bCTCs and bCSCs are rare in blood, they play a key role in tumor metastasis. Detection of CTCs and CSCs can be used as a non-invasive "liquid biopsy" for tumor diagnosis and prognosis. Glycan biomarkers of bCTCs and bCSCs have unique value in BCa healthcare, especially in personalized therapy that targets specific immunotypes of BCa. Thus, a practical strategy to facilitate identification and characterization of potential glycan biomarkers of bCTC and bCSC, as described in accordance with various embodiments, is beneficial.

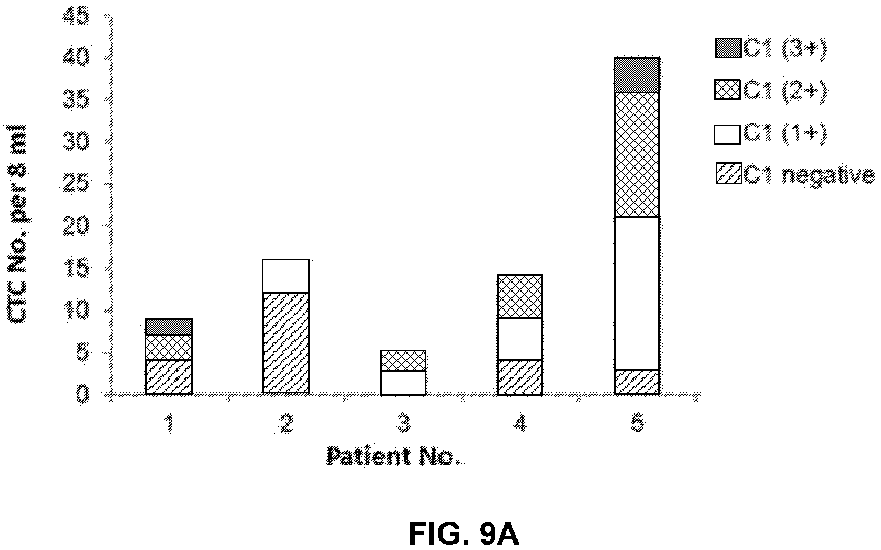

In various experimental embodiments, blood samples from five Stage IV metastatic breast cancer (MBCA) patients are characterized (as further illustrated and described in connection with FIGs. 9A-9B). Glycan biomarker gpC1 positive CTCs are detected in all subjects; approximately 40% of bCTCs are strongly gpC 1 positive. Interestingly, the CTCs from a triple-negative breast cancer (TNBC) patient with multiple sites of metastasis are predominantly gpC1 positive (92.5%, 37/40 CTCs). This demonstrates the feasibility of detecting CTC-glycan biomarkers using FAST-scan technology.

Cell-surface expression of gpC1 in a panel of tumor cell lines is also characterized, in specific embodiments, by a glycan-specific flow cytometry assay. In a first set of experiments, tumor cell lines of distinct tissue origin, including a BCA line, T-47D; a lung cancer (LCA) line, A549; a prostate cancer (PCA) line, PC3; and a skin-derived melanoma cell line, SKMEL-28, are examined. SKMEL-28 (melanoma) and PC3 (PCA) are negative, A549 (LCA) are weakly positive, but T-47D (BCA) are strongly positive. In the second set of staining, a panel of seven human BCA lines are examined. These include two estrogen-receptor-positive (ER+) and progesterone-receptor-positive (PR+) lines (T-47D and MCF-7), one ER+ (SK-BR-3), and four triple-negative (TN) cancers that lacked the estrogen, progesterone, and Her2)/neu receptors (BT-549, Hs 578T, MDA-MB-231, and MDA-MB-468). T-47D and MCF-7 are strongly positive, and SK-BR-3 are intermediately positive in gpC1 expression. Notably, two TNBC lines, BT-549 and MDA-MB-468, are found to be strongly gpC1 positive. In contrast, the two remaining TNBC lines, Hs578T and MDA-MB-231, are negative.

Somewhat surprisingly, some BCA cell lines analyzed exhibit CSC-phenotype and potency in establishing a metastatic tumor in vivo. The TNBC line MDA-MB-231 is phenotypically CD44+/CD24- and is able to establish bone metastasis in nude mice. MDA-MB-468 is phenotypically CD44+/CD24+ and is highly efficient in lung (but not bone) metastasis. MDA-MB-231 is gpC1-negative but the lung metastatic MDA-MB-468 and another TNBC line BT-549 are strongly positive in gpC1-expression. These findings shed light on the glycomics diversity of bCTCs/CSCs.

An anti-tumor glycan mAb C1 serves as a key reference reagent for monitoring tumor cell-surface expression of gpC1. Of note, the parental hybridoma cell line of C1, called HAE3, is raised against epiglycanin, the major sialomucin glycoprotein (around 500 kDa) of murine mammary adenocarcinoma TA3 cells. Additionally, this anti-murine carcinoma antibody exhibits strong cross-reactivity with a number of human epithelial tumors in tissues, including lung, prostate, bladder, esophageal, and ovarian cancers. This cross-species tumor-binding profile indicates antibody recognition of a conserved tumor glycan biomarker that is co-expressed by both mouse- and human-derived epithelial cancers.

Carbohydrate microarrays are introduced to explore the potential natural ligands of C1 and HAE3. For this purpose, a large collection of purified natural carbohydrate antigens are applied for the microarray screening. A number of blood group substances are spotted in this carbohydrate microarray together with a large collection of carbohydrate antigens to examine the antibody binding specificity. The antibodies C1 and HAE3 selectively bond to a number of blood-group precursor antigens. These precursor substances are prepared to remove and are essentially devoid of most of the α-L-fucosyl end groups that are essential for blood group A, B, H, or Lewis active side chains but possess the internal domains or core structures of blood group substances. By contrast, these mAbs have no or minimal detectable cross-reactivity with blood group substances A, B, O, or Lewis antigens, or the large panel of other carbohydrate antigens spotted in the same array.

Selective detection of these blood group precursors from a large panel of blood group substances by the mAbs illustrate they are specific for a shared cryptic glyco-epitope of these precursor substances. This microarray finding is further validated by glycan-specific enzyme-linked immunosorbent assay (ELISA) and glyco-conjugate-based epitope competition assays.

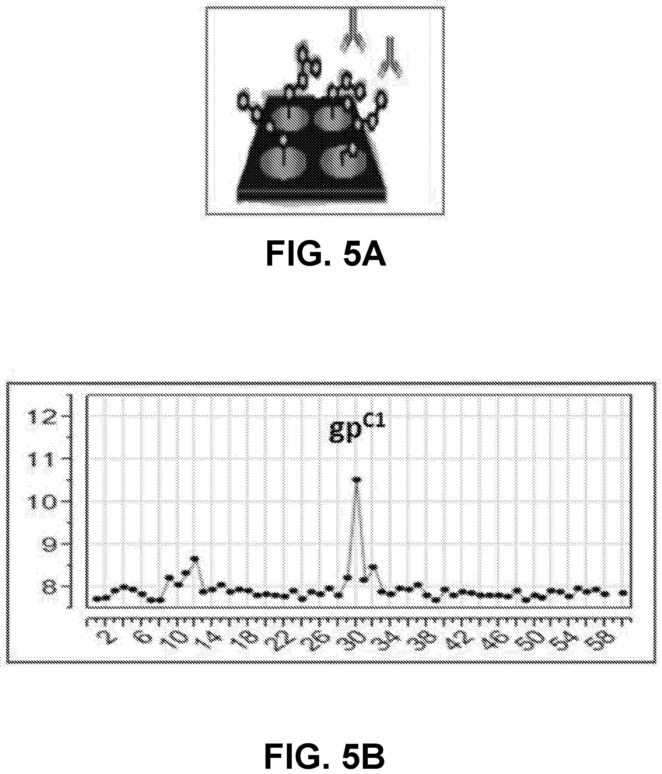

FIGs. 5A-5D illustrate example tools used to identify glycan biomarkers of CTC and CSCs, in accordance with various embodiments. FIG. 5A illustrates an example of a glycan-array, in accordance with various embodiments, and as further described below. FIG. 5B illustrates an example of a glycan biomarker, in accordance with various embodiments.

FIG. 5C illustrates example optical circuitry, in accordance with various embodiments. The optical circuitry illustrated is a fiber optic scanner 550. The fiber optic scanner 550 can be a portion of an apparatus, such as the apparatus 102 illustrated in FIG. 1. The fiber optic scanner 550 can be in communication with processing circuitry to form an apparatus that can identify CSCs from a blood sample and can profile the cell population of the blood sample.

The fiber optic scanner 550 includes a light source (e.g., laser 556) to excite fluorescence located in a sample. The sample (e.g., blood sample) is immobilized or fixed to a substrate and can be held in place by a stage 553. In specific embodiments the light source is a laser 556, such as a 10 mW Argon laser that can excite fluorescence in labeled cells. The fluorescence can be collected in optics with a large (e.g., 50 mm) field-of-view. The field-of-view is enabled by an optic fiber bundle 554. The optic fiber bundle 554 can have asymmetric ends, in some embodiments, and the resolution of the fiber optic scanner 550 can be determined by the spot size of the light source. The emissions from the fluorescent probe can be filtered through dichroic filters before detection at imaging circuitry 557, such as a photomultiplier. The substrate, via the stage 553, can be moved orthogonally across the light scan path on the stage 553. The location of a fluorescently labeled cell is determined by the scan and the stage positions at the time of emission (and to an accuracy of ± 70 um). For more specific and general information regarding an example FAST system, reference is made to Hsieh HB, Marrinucci D, Bethel K, et al., "High speed detection of circulating tumor cells", Biosensors and Bioelectronics, 2006; 21: 1893-1899, and Krivacic RT, Ladanyi A, Curry DN, et al., "A rare-cell detector for cancer", Proc Natl Acad Sci USA. 2004;101: 10501-10504.

The fiber optic scanner 550 illustrated can include a FAST system as implemented by SRI International, however embodiments are not so limited and other high speed scanning methods such as multispectral or hyperspectral imaging may be used. FAST was originally developed for the rapid detection of CTCs, including enabling high throughput scanning for fluorescently-labeled CTCs. Briefly, blood collected from patient and the red blood cells are lysed, and white cells are adhered and fixed to a pretreated glass slide and permeabilized for immunofluorescent labeling. After labeling, the slide is scanned, such as using laser-printing optics, an array of optical fibers that detects fluorescence emission from the cells.

CSCs and/or CTCs are considered the seeds of residual disease and distant metastases, and their characterization are useful for early detection biomarkers, and may guide treatment options. FAST technology is a high-throughput, high-sensitivity scanner to scan all nucleated cells for an unbiased detection of CSCs and/or CTCs on a planar substrate. The instrument enables rapid location of CSCs and/or CTCs without the need for special enrichment, so its sensitivity is not degraded through, e.g., EpCAM targeted antibody enrichment. Because the sample preparation protocol does not distort cell morphology, CSCs and/or CTCs are located on a planar surface and CSC and/or CTC imaging is of high fidelity, which leads to improved specificity. FAST also enables the simultaneous (multiplexed) analysis of multiple protein, cytogenetic, and molecular biomarkers at a single CTC and CSC level.

Once the cancerous (CSC or CTC tumor) cells are identified, further investigation can examine the characteristics of single cells by immunohistochemistry and other analyses such as fluorescence in situ hybridization (FISH), polymerase chain reaction (PCR), and single nucleotide polymorphism (SNP) analysis.

In various embodiments not encompassed by the present invention, the apparatus including the fiber optic scanner 550 and the processing circuitry can include additional circuitry. For example, the apparatus can include a server for storage of data sets, internal network connecting instrumentation control and database, and computer software for instrument control and data management (sometimes herein referred to as "processing circuitry" for ease of reference).

FIG. 5D illustrates an example of a resulting immunotype of CTCs and CSCs from a scan of blood samples using optical circuitry, in accordance with various embodiments. The gpC1 positive CTCs are stained in green in the background of the DAPI-blue labeling of white blood cells (e.g., large circles in the top of FIG. 5D) and co-stained by an anti-cytokeratin (CK) antibody in red (e.g., the large circles in the bottom of FIG. 5D).

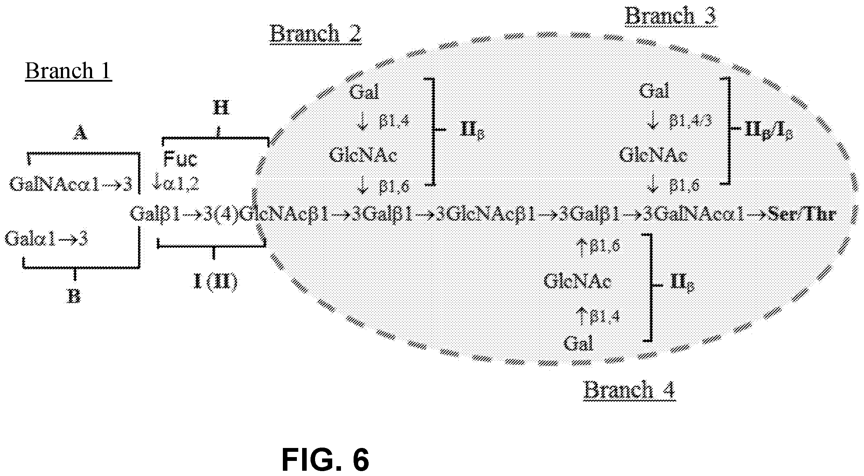

FIG. 6 illustrates an example of a blood group substance with a conserved O-glycan core, in accordance with various embodiments. More specifically, FIG. 6 illustrates the common blood group precursor core structure highlighted. The four types of branched structures illustrate the potential complexity of the internal portion of the carbohydrate moiety of blood group substances, which is proposed based on extensive immunochemical characterization of blood group substances.

As previously described, the glycan biomarker includes at least one chain selected from the group consisting of polylactosamine chains, oligosaccharide chains, and combinations thereof. The at least one chain has branches selected from the group consisting of IIβ (Galβ1,4GlcNAcβ1,6), Iβ(Galβ1,3GlcNAcβ1,6), IIβ/Iβ (Gal β1, 4/3GlcNAc β1,6) -moieties, and combinations thereof.

In some specific embodiments, gp^{C1}-based blood group precursor epitopes are characteristically composed of a plurality of oligosaccharide chains with branches of IIβ (Galβ1,4GlcNAcβ1,6) and/or Iβ (Galβ1,3GlcNAcβ1,6) moieties without fucosylation. The fucosyl epitopes are essential for forming blood group A, B, H, or Le antigens; the terminal nonreducing β-galactoside epitopes of Iβ and/or IIβ are crucial for preserving the conserved gpC1 epitope (s) of BCA. The internal chain of blood group precursor may also express targetable biomarkers.

Tumor-associated overexpression of blood-group-related autoantigens is not limited to BCA. As recently reported, the natural ligand of a PCA-specific mAb F77 is blood group H, which is built on a 6-linked branch of a poly-N-acetyllactosamine backbone. Overexpression of gpF77 in PCA may reflect increased blood group H expression together with up-regulated expression of branching enzymes. HAE3 and C1 differ from F77 in glycan binding specificities and tumor-binding profiles. Unlike F77, which is blood-group-H specific and stains the PCA cell line PC3, HAE3 and C1 have neither reactivity with blood group H nor the cell surface targets of PC3. Taken together, these illustrate that epithelial tumor expression of blood-group substance-related autoantigens. This further illustrates the potential of this class of carbohydrate-based immunological targets for tumor vaccine development and targeted immunotherapy.

### Materials and Methods

### Patient Samples

CTCs analyzed were from patients undergoing treatment for metastatic breast cancer at the City of Hope Cancer Center. Blood samples were collected and used under protocols approved by the Institutional Review Boards of the City of Hope Cancer Center, Palo Alto Research Center, and SRI International. All patients gave their written, voluntary, informed consent (www.clinicaltrials.gov: NCT01048918 and NCT00295893). Patient demographics and clinical characteristics are described in the results section.

**Table 1. Dataset from a Carbohydrate Microarray Analysis of MAb C1**

| **Carbohydrate antigens** | | **Fluorescence intensity (INT)** | | | | **Microarray scores (Log2-INT)** | | |
|---|---|---|---|---|---|---|---|---|
| **Concentrations (µg/µl)** | **ID#** | ***N*** | **Mean** | **StDev** | **^{a}Ag./Bg.** | **Mean** | **StDev** | ***t-Test* (*p* value)** |
| Man2-PAA (0.002) | 1 | 6 | 237 | 31 | 1.00 | 7.8737 | 0.1803 | 0.914545639 |
| Man2-PAA (0.01) | 2 | 6 | 231 | 19 | 0.98 | 7.8443 | 0.1178 | 0.555761263 |
| Man2-PAA (0.05) | 3 | 6 | 239 | 30 | 1.01 | 7.8892 | 0.1736 | 0.956085932 |
| Man2-PAA (0.25) | 4 | 6 | 265 | 20 | 1.12 | 8.0462 | 0.1106 | 0.032956982 |
| Man5-BSA (0.002) | 5 | 6 | 248 | 10 | 1.05 | 7.9536 | 0.0576 | 0.088125398 |
| Man5-BSA (0.01) | 6 | 6 | 237 | 15 | 1.00 | 7.8824 | 0.0873 | 0.97480637 |
| Man5-BSA (0.05) | 7 | 6 | 232 | 27 | 0.98 | 7.8443 | 0.1619 | 0.687266729 |
| Man5-BSA (0.25) | 8 | 6 | 234 | 24 | 0.99 | 7.8572 | 0.1563 | 0.786298278 |
| ***^{b}Man9-BSA*** (0.002) | ***9*** | ***6*** | ***321*** | ***25*** | ***1.36*** | ***8.3162*** | ***0.1109*** | ***0.000876789*** |
| ***Man9-BSA*** (0.01) | ***10*** | ***6*** | ***286*** | ***20*** | ***1.21*** | ***8.1551*** | ***0.1009*** | ***0.00212074*** |
| ***Man9-BSA*** (0.05) | ***11*** | ***6*** | ***320*** | ***17*** | ***1.35*** | ***8.3180*** | ***0.0782*** | ***3.98574E-06*** |
| ***Man9-BSA*** (0.25) | ***12*** | ***6*** | ***387*** | ***36*** | ***1.64*** | ***8.5863*** | ***0.1348*** | ***6.48251E-05*** |
| P-Man (0.002) | 13 | 6 | 245 | 15 | 1.04 | 7.9332 | 0.0870 | 0.353052118 |
| P-Man (0.01) | 14 | 6 | 252 | 15 | 1.07 | 7.9755 | 0.0872 | 0.115549225 |
| P-Man (0.05) | 15 | 6 | 259 | 20 | 1.10 | 8.0088 | 0.1125 | 0.093583925 |
| P-Man (0.25) | 16 | 6 | 259 | 29 | 1.10 | 8.0016 | 0.1430 | 0.258706274 |
| LAM (0.002) | 17 | 6 | 242 | 10 | 1.03 | 7.9172 | 0.0596 | 0.393531249 |
| LAM (0.01) | 18 | 6 | 239 | 14 | 1.01 | 7.8944 | 0.0817 | 0.83690219 |
| LAM (0.05) | 19 | 6 | 251 | 27 | 1.06 | 7.9600 | 0.1531 | 0.423657402 |
| LAM (0.25) | 20 | 6 | 240 | 23 | 1.02 | 7.9004 | 0.1399 | 0.821364692 |
| OR (0.05) | 21 | 6 | 220 | 23 | 0.93 | 7.7703 | 0.1495 | 0.200031297 |
| OR (0.25) | 22 | 6 | 225 | 13 | 0.95 | 7.8081 | 0.0828 | 0.166498725 |
| ASOR (0.05) | 23 | 6 | 233 | 20 | 0.99 | 7.8605 | 0.1242 | 0.714768394 |
| ASOR (0.25) | 24 | 6 | 219 | 16 | 0.93 | 7.7697 | 0.1048 | 0.09026433 |
| AGOR (0.05) | 25 | 6 | 230 | 15 | 0.98 | 7.8412 | 0.0916 | 0.493370387 |
| AGOR (0.25) | 26 | 6 | 232 | 12 | 0.98 | 7.8553 | 0.0759 | 0.59077204 |
| Tij II (0.002) | 27 | 6 | 240 | 17 | 1.02 | 7.9012 | 0.0997 | 0.784186197 |
| Tij II (0.01) | 28 | 6 | 247 | 27 | 1.04 | 7.9331 | 0.1558 | 0.594715802 |
| ***Tij II*** (0.05) | ***29*** | ***6*** | **326** | ***30*** | ***1.38*** | ***8.3403*** | ***0.1354*** | ***0.001294538*** |
| ***Tij II*** (0.25) | ***30*** | ***6*** | ***1908*** | ***409*** | ***8.08*** | ***10.8562*** | ***0.3151*** | ***5.40788E-06*** |
| ***aGal-BSA*** (0.05) | ***31*** | ***6*** | ***284*** | ***17*** | ***1.20*** | ***8.1450*** | ***0.0864*** | ***0.000573103*** |
| ***aGal-BSA*** (0.25) | ***32*** | ***6*** | ***359*** | ***25*** | ***1.52*** | ***8.4831*** | ***0.0998*** | ***7.30853E-06*** |
| IM3-BSA (0.002) | 33 | 6 | 251 | 13 | 1.06 | 7.9660 | 0.0699 | 0.144584279 |
| IM3-BSA (0.01) | 34 | 6 | 239 | 19 | 1.01 | 7.8940 | 0.1198 | 0.877710292 |
| IM3-BSA (0.05) | 35 | 6 | 262 | 21 | 1.11 | 8.0259 | 0.1133 | 0.071497953 |
| IM3-BSA (0.25) | 36 | 6 | 253 | 12 | 1.07 | 7.9773 | 0.0742 | 0.113684458 |
| LD7 (0.02) | 37 | 6 | 241 | 27 | 1.02 | 7.9002 | 0.1542 | 0.85762395 |
| LD7 (0.1) | 38 | 6 | 237 | 12 | 1.00 | 7.8843 | 0.0732 | 0.994141248 |
| B1299S (0.02) | 39 | 6 | 232 | 26 | 0.98 | 7.8470 | 0.1604 | 0.657503756 |
| B1299S (0.1) | 40 | 6 | 240 | 17 | 1.02 | 7.9012 | 0.1008 | 0.768999737 |
| B1355S (0.02) | 41 | 6 | 232 | 18 | 0.98 | 7.8517 | 0.1077 | 0.599367191 |
| B1355S (0.1) | 42 | 6 | 241 | 20 | 1.02 | 7.9074 | 0.1182 | 0.729147575 |
| Dex2000K (0.02) | 43 | 6 | 244 | 14 | 1.03 | 7.9277 | 0.0829 | 0.431623112 |
| Dex2000K (0.1) | 44 | 6 | 237 | 16 | 1.00 | 7.8816 | 0.0986 | 0.965736843 |
| N279 (0.02) | 45 | 6 | 218 | 20 | 0.92 | 7.7614 | 0.1345 | 0.134677298 |
| N279 (0.1) | 46 | 6 | 247 | 24 | 1.05 | 7.9343 | 0.1270 | 0.588883272 |
| Levan (0.02) | 47 | 6 | 239 | 25 | 1.01 | 7.8887 | 0.1590 | 0.95570906 |
| Levan (0.1) | 48 | 6 | 240 | 16 | 1.02 | 7.9032 | 0.0922 | 0.740106425 |
| E. coli 0111:B4 (0.02) | 49 | 6 | 224 | 30 | 0.95 | 7.7952 | 0.1949 | 0.38369683 |
| E. coli 0111:B4 (0.1) | 50 | 6 | 232 | 16 | 0.98 | 7.8516 | 0.0984 | 0.570865809 |
| E. coli K92 (0.02) | 51 | 6 | 233 | 16 | 0.99 | 7.8608 | 0.0976 | 0.683969572 |
| E. coli K92 (0.1) | 52 | 6 | 245 | 15 | 1.04 | 7.9347 | 0.0884 | 0.359544745 |
| K. pneumoniae (0.02) | 53 | 6 | 237 | 24 | 1.01 | 7.8800 | 0.1486 | 0.962786162 |
| K. pneumoniae (0.1) | 54 | 6 | 238 | 19 | 1.01 | 7.8888 | 0.1210 | 0.94198138 |
| S. typhi (0.02) | 55 | 6 | 247 | 22 | 1.05 | 7.9424 | 0.1222 | 0.411190903 |
| S. typhi (0.1) | 56 | 6 | 243 | 19 | 1.03 | 7.9146 | 0.1081 | 0.666740193 |
| PnSIV (0.02) | 57 | 6 | 231 | 23 | 0.98 | 7.8408 | 0.1457 | 0.603461225 |
| PnSIV (0.1) | 58 | 6 | 234 | 17 | 0.99 | 7.8625 | 0.1087 | 0.740270948 |
| Background | | 96 | 236 | 23 | | 7.8840 | 0.1522 | |

| | | | | | | | | |
|---|---|---|---|---|---|---|---|---|
| ^{a}Ag./Bg: Ratio of mean fluorescence intensity of antigen and mean background. ^{b}Positive detections are highlighted with bold italics. A positive score is given if the mean fluorescence intensity value of an antibody activity for a given antigen preparation (antigen spot# = 6) is significantly higher than the mean of background spots (n = 96) with a p-value < 0.01. | | | | | | | | |

### Carbohydrate Antigens, Anti-glycan Antibodies, and Tumor Cell Lines

Carbohydrate antigens for carbohydrate microarray analysis are listed in Table 1. Antibody C1 (IgM) is produced in specific experiments by cell line HAE3-C1, which is a subclone of the parent murine hybridoma, HAE3. Tumor cell lines used include lung (A549)- and breast (T47D and SKBR3)-derived epithelial tumor cell lines. Both T47D and SKBR3 are derived from metastatic sites in breast cancer patients. All tumor cell lines are acquired from the American Type Culture Collection (ATCC), Manassas, VA.

Antigen preparations and key references: Man2-PAA: Manα1,2Man-polyacrylamide, this report; Man5-BSA: (Man5GlcNAc2Asn)n-BSA, Wang, et al., Drug Dev Res. 75, 172 (2014); Man9-BSA: (Man9GlcNAc2Asn)n-BSA, Wang, et al., Drug Dev Res. 75, 172 (2014); P-Man: Yeast phosphomannan polysaccharide, NRRL B-2448, Kabat et al., J. Exp. Med. 164, 642 (1986); LAM: Lipoarabinomannan from Mycobacterium tuberculosis Aoyama-B, this report; OR: Orosomucoid (α1-acid glycoprotein), Wang and Lu, Physiol Genomics 18(2), 245 (2004); ASOR: Asialo-orosomucoid-expressing Tri/m-II glyco-epitopes, Wang & Lu, Physiol Genomics 18(2), 245 (2004); AGOR: Agalacto-orosomucoid-expressing Tri/m-Gn glyco-epitopes, Wang & Lu, Physiol Genomics 18(2), 245 (2004); Tij II: Blood group precursor Tij II 20%fr. 2nd 10%, Maisonrouge-McAuliffe & Kabat, Arch. Biochem. Biophys. 175, 71(1976); αGal-BSA: Galα1,3Galβ1, 4Glcβ1-BSA, this report; IM3-BSA: Isomaltotriose-BSA, Zopf et al., Methods Enzymol. 50, 163 (1978); LD7: α(1→6)dextran, Linear chains, Wang et al., Nat Biotechnol 20(3):275-81, (2002); B1299S: α(1→6)dextran, NRRL B-1299S, Wang et al., Nat Biotechnol 20(3):275-81, (2002); B1355S: α(1→3)(1→6)dextran, NRRL B-1355S, Wang et al., Nat Biotechnol 20(3):275-81, (2002); Dex-2000K: Fluorescein isothiocyanate-conjugated dextrans (2,000 kDa), Wang et al., Nat Biotechnol 20(3):275-81, (2002); N279: α(1→6)dextran, NRRL N279, Wang et al., Nat Biotechnol 20(3):275-81, (2002); Levan: Levan purified from preparation of B-512-E dextran, Kabat et al., J. Exp. Med. 164, 642 (1986); E. coli 0111:B4: Lipopolysaccharides from Escherichia coli O111:B4 L 2630, Sigma-Aldrich Co., St Louis, MO; E. coli. K92: E. coli K92 polysaccharide, Kabat et al., J. Exp. Med. 164, 642 (1986); K. pneumoniae: Lipopolysaccharides from Klebsiella pneumoniae L4268, Sigma-Aldrich Co., St Louis, MO; S. typhi LPS: Lipopolysaccharides from Salmonella enterica serotype typhimurium L7261, Sigma-Aldrich Co., St Louis, MO; Pn SIV: Pneumococcus type IV soluble polysaccharide, Kabat et al., J. Exp. Med. 164, 642 (1986); OG: Blood group precursor substance OG 10% 2X, Vicari & Kabat, J. Immunol. 102, 821 (1969); Feizi et al., J. Exp. Med. 133, 39 (1971); EPGN: Asialo-epiglycanin, produced by murine mammary adenocarcinoma TA3 cells, Codington et al., Biochemistry, 11, 2559 (1972).

### Carbohydrate Microarrays and ELISA

Carbohydrate antigens of various structural compositions are dissolved in phosphate-buffered saline (PBS) (glycoprotein conjugates) or saline (polysaccharides) and spotted onto SuperEpoxy 2 Protein slides (Array It Corporation, Sunnyvale, CA) by a high-precision robot designed to produce cDNA microarrays (Cartesian Technologies PIXSYS 5500C). Immediately before use, the printed microarray slides are washed in 1× PBS at room temperature for 5 minutes and blocked with 1% bovine serum albumin (BSA)-PBS at room temperature for 30 minutes. They are incubated at room temperature with C1 (IgM) antibody at 5.0 µg/mL in 1% (wt/vol) BSA in PBS containing 0.05% (wt/vol) NaN3 and 0.05% (vol/vol) Tween 20. An R-phycoerythrin (R-PE)-conjugated affinity-purified F(ab') fragment of goat anti-mouse IgM secondary antibody preparation (Rockland Immunochemicals, Inc., Limerick, PA) is applied at 2.0 µg/mL to reveal the C1-specific staining signal. The stained slides are rinsed five times with PBS with 0.05% (vol/vol) Tween 20, air-dried at room temperature, and then scanned for fluorescent signal using a ScanArray5000A Microarray Scanner (PerkinElmer Life Science). The SAS Institute JMP-Genomics software package (http://www.jmp.com/) is used for further microarray data standardization and statistical analysis. Results of the microarray assay are shown as microarray scores, i.e., means of the log2-transformed fluorescent intensities (MFIs) of multiple detections of a given antigen preparation. Glycan-specific ELISA can also be performed following a standard protocol.

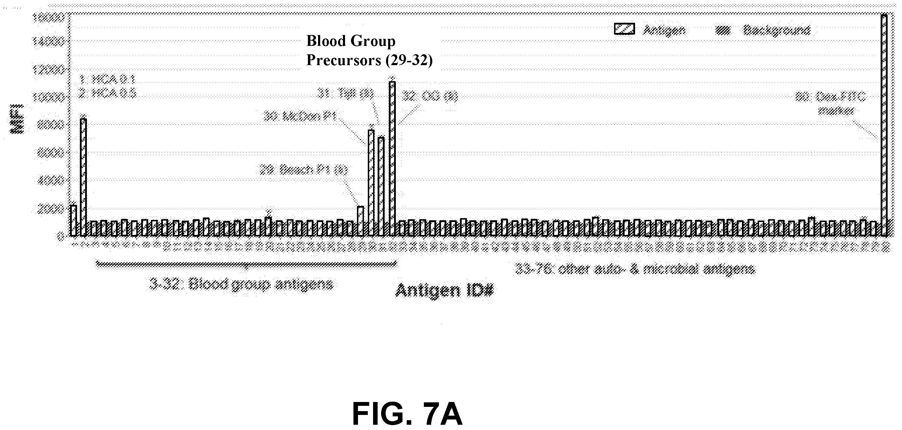

FIG. 7A-7B illustrates example experimental results of a carbohydrate microarray used to identify glycan biomarkers using antibody HAE3, in accordance with various embodiments. As previously described, a large collection of purified natural carbohydrate antigens are applied for carbohydrate microarray construction. Blood group substance reference reagents (Kabat 1956) used include Cyst 9 and Cyst 14, A active; Beach phenol insoluble, B active; Hog, H active; JS phenol insoluble, H and Leb active, and N-1 20% from the second 10%, Lea active. A number of blood group precursor references, including OG, Tij II, Beach P1, and McDon P1 (29#-32#), are spotted in this carbohydrate microarray. These precursor substances are prepared to remove most of the α-L-fucosyl end groups that are essential for blood group A, B, H, or Lewis active side chains, but possess the internal domains or core structures of blood group substances. A large panel of other autoantigens and microbial polysaccharides are also spotted in the same microarrays to critically examine the antibody binding specificity. A preparation of HAE3-reactive human carcinomaassociated antigen (HCA) serves as a positive control for this assay (Li et al. 2004).

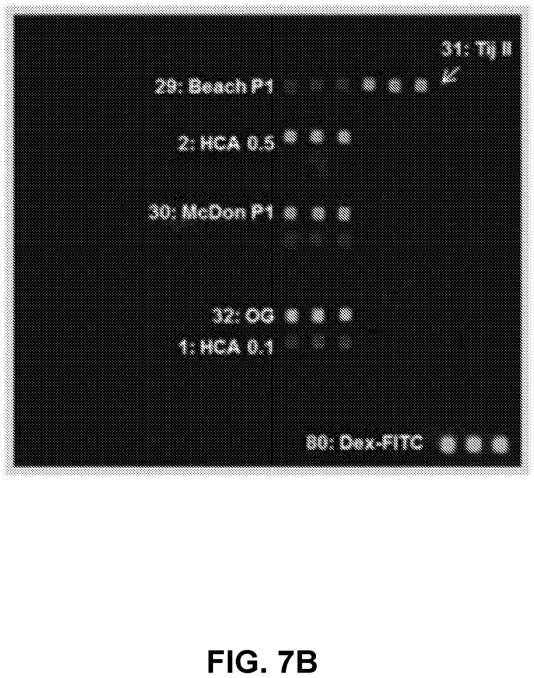

FIG. 7A is a graph illustrating the HAE3 binding signal (red column) as plotted with corresponding local background reading (blue column) as an overlay plot. Each data point represents the mean of triplicate detections; these are shown in the FIG. 7B microarray image with the number of positive antigens labeled. Each error bar is constructed using one standard deviation from the mean. As illustrated, HAE3 is strongly positive with HCA (1# and 2#) as expected. This antibody selectively binds to four blood group precursor antigens, Beach P1 (29#), McDon P1 (30#), Tij II (31#), and OG (32#). By contrast, HAE3 has no detectable crossreactivity with blood group substances A, B, O, or Lewis antigens, or the large panel of other carbohydrate antigens spotted in the same array.

### Flow Cytometry Analysis to Examine Tumor Cell Surface Expression of HAE3⁺ Glyco-Epitopes

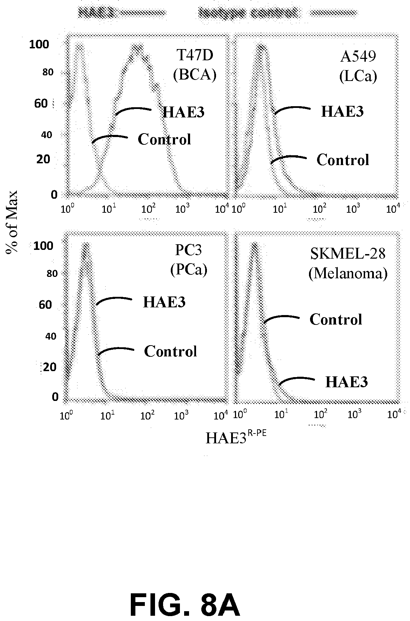

FIGs. 8A-8B illustrate example experimental results showing expression of surface tumor biomarkers, in accordance with various experimental embodiments. As illustrated by FIGs. 8A-8B, various experimental embodiments further examined whether the HAE3+ glyco-epitopes are expressed as cell-surface tumor biomarkers. To ensure the observed cross-species antigenic reactivities are not owing to the unexpected presence of oligoclonal populations in the original HAE3 hybridoma cell line, HAE3 is subcloned to produce an antibody from a single clone, HAE3-C1 (C1). Antibody C1 is verified by carbohydrate microarrays and a glycan-specific ELISA to be highly specific for a conserved *O*-glycan cryptic glyco-epitope gpC1 in human blood group precursors.

In an example set of experiments, a panel of four tumor cell lines are screened by cell surface staining in flow cytometry. These include (a) a BCa line, T-47D, which is selected owing to the fact that breast cancer patients are found to produce substances in circulation that are highly effective in inhibiting AE3-binding of epiglycanin; (b) a lung cancer (LCa) line, A549, which is known to produce an HAE3-positive substance in cell culture; (c) a prostate cancer (PCA) line, PC3, which is found to express a blood group B-related F77 glyco-epitope; and (d) a melanoma cell line SKMEL-28, which is derived from skin but not epithelial tissue.

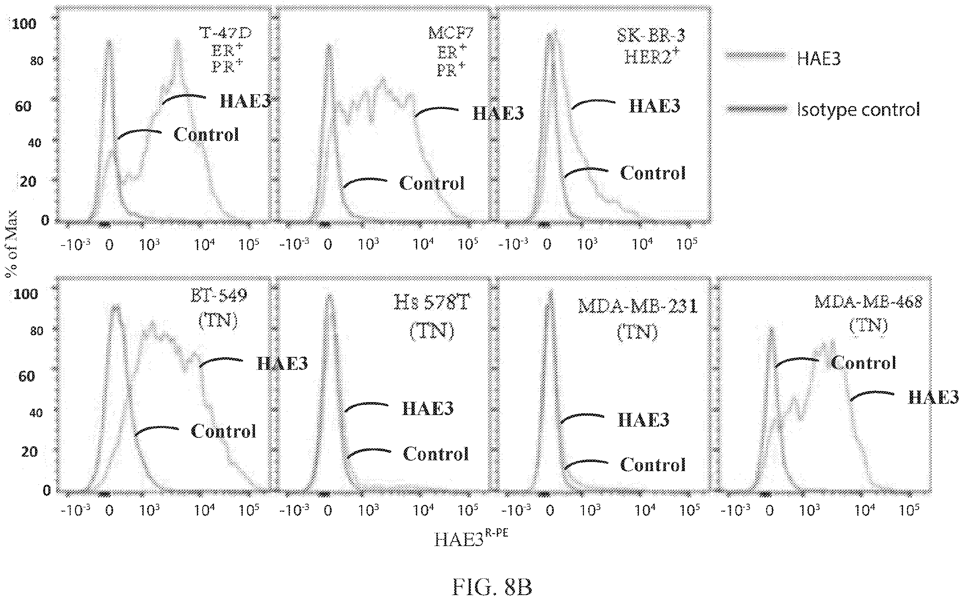

As shown in FIG. 8A, melanoma SKMEL-28 and prostate cancer PC3 are negative for HAE3. The A549 lung cancer cell line was weakly positive. By contrast, the breast cancer cell line T-47D are strongly positive in HAE3-cell surface staining. Given these results, the flow cytometry analysis is extended to a panel of seven human breast cancer cell lines, including two estrogen receptor positive (ER+) and progesterone receptor positive (PR+) lines (T-47D and MCF-7), one ER+ (SK-BR-3), and four triple-negative (TN) cancers that lack the estrogen, progesterone, and Her2)/neu receptors (BT-549, Hs 578 T, MDA-MB-231, and MDA-MB-468). FIG. 8B shows that two ER+PR+ lines, T-47D and MCF-7, and two triplenegative lines, BT-549 and MDA-MB-468, are HAE3 strongly positive. SK-BR-3 is intermediately positive. By contrast, the two remaining triple-negative cell lines, Hs578T and MDA-MB-231, are HAE3 negative.

### Detection of Glycan Biomarker-Positive bCTC/CSC in Stage IV Breast Cancer Patients

With antibody C1 as a key probe, it can be determined that gpC1 is applicable for detection and immunotyping analysis of CTCs/CSCs in patients with metastatic breast cancer. In a pilot clinical case study, blood samples from five Stage IV breast cancer patients are characterized the FAST-scan technology.

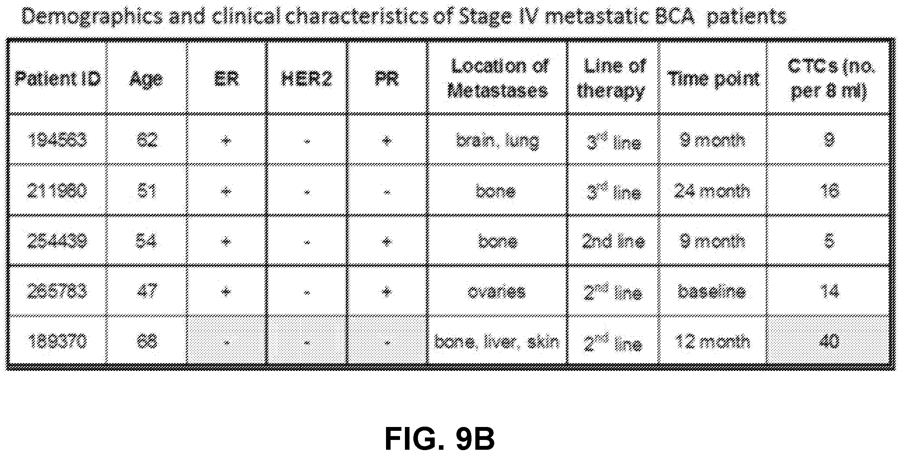

FIGs. 9A-9B illustrate example experimental results from characterizing blood samples from five Stage IV breast cancer patients, in accordance with various embodiments. As previously illustrated by FIG. 5D, CTCs captured from the Stage IV breast cancer patients can be scored as 3+, 2+, 1+, and 0, left to right in the graph. Four representative bCTCs are shown in which the epithelial-derived cells are labeled by anti-cytokeratins (CK) antibodies in red, and the gpC1 positive cells are stained in green in the background of the DAPIblue labeling of white blood cells. FIG. 9A and FIG. 9B show that all subjects characterized have gpC1-positive CTCs. Approximately 40% of CTCs captured in these patients express higher levels (2+ and 3+) of the gpC1 biomarkers; gpC1- positive and - negative CTCs are found to co-exist in four subjects. Notably, a triple-negative patient (ID# 189370) produced predominantly gpC1-positive CTCs (37 of 40 CTCs) with 50% scored gpC1 2+/3+. In this patient, metastatic tumors are seen in multiple sites, including bone, liver, and skin, which is indicative of the presence of gp^{C1+} CSCs in the CTCs population.

### Carbohydrate Microarrays Detect Glyco-Determinants in Human Carcinoma-associated Antigen (HCA)

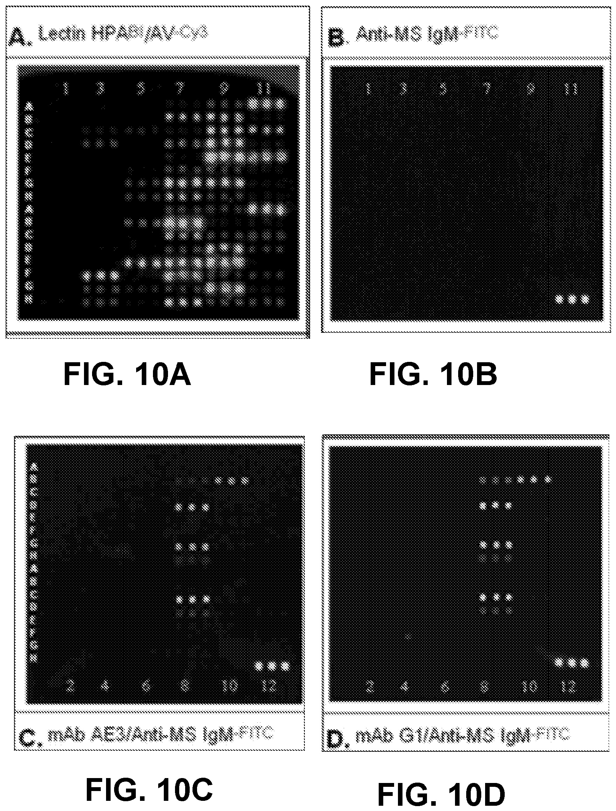

FIGs. 10A-10D illustrate example experimental results of use of carbohydrate microarrays for detecting glycan biomarkers using antibodies G1 and HAE3, in accordance with various embodiments. As shown in FIGS. 10A-10D and Table 2, mAb G1 and AE-3 are highly reactive with three glycoconjugates (ovarian cyst glycoproteins) designated Beach P1 insol., Tij 2 20% fr. 10%, and Ogunsheye 10% 2x but that they are marginally reactive with neoglycoconjugates that display T and Tn glyco-epitopes. The antibody binding signals elicited by these three glycoproteins are in the range elicited by HCA and the original immunogen, epiglycanin (EPGN). Thus, G1 and AE-3 are specific for the glyco-epitopes that are shared among the five glycoproteins. The three strongly positive ovarian glycoproteins are known for their expression of antigenicities associated with the *O*-glycan core and backbone domains such as Ii antigens, or Lewis antigens. Therefore, G1 and AE-3 are directed to glyco-epitopes associated with the complex backbone sequences rather than T/Tn structures as previously postulated. This blood group precursor epitope is referred to herein as gpC1 and/or gp^{C1}.

More specifically, FIGs. 10A-10D illustrate results from a set of carbohydrate microarrays used to examine the binding of anti-HCA mAb G1 and HAE3, sometimes interchangeably referred to as AE3. Forty eight glycoproteins and neoglycoconjugates are spotted in triplicates and in two dilutions to yield a total of 288 microspots per microarray slide. Images of microarrays stained with: lectin Helix pomatia agglutinin (HPA), as illustrated by FIG. 10A, which is highly cross-reactive with Gal/GalNAc-terminated glyco-epitopes and serves as a reagent for monitoring efficacy of immobilization of Gal-containing glycoconjugates; Anti-mouse IgM alone, as illustrated by FIG. 10B; mAb HAE3, as illustrated by FIG. 10C; and G1, as illustrated by FIG. 10D.

Table 2, as illustrated below, shows relative reactivities of mAb HAE3 and G1 with blood group substances and their precursors, which is measured as ratio of mean fluorescent intensity to mean background. The positive reactivities are highlighted in bold. MAb G1 and HAE3 were provided by Egenix, Inc. (Rochester, NY). In Table 2, only 12 of the glycoconjugates tested are shown as the rest are negative as statistically measured with cutoff of around ratio 1.5, illustrating the specificities of G1 and HAE3.

**Table 2: Carbohydrate Microarray Characterization of Blood Group Substance Binding Reactivities of Anti-HCA mAbs HAE3 and G1.**

| **Carbo-microspots** | | | | **Anti-MS IgM** | | **mAb AE3/Anti-MS IgM** | | **mAb G1/Anti-MS IgM** |
|---|---|---|---|---|---|---|---|---|
| **Glycoconjugates** | **Class** | **ID** | | **Int./Bk.*** | | **Int./Bk.** | | **Int/Bk.** |
| N-1 10% 2X | Le^{a} | B-5 | | 1.17 | | 1.14 | | 1.14 |
| N-1 IO4 NaOH | Le^{a} | B-6 | | 1.15 | | 1.16 | | 1.16 |
| Beach P1 φ insol. | B, Ii | B-7 | | 1.17 | | **2.11** | | **3.46** |
| Cyst 9 φ sol. | A | B-8 | | 1.16 | | 1.17 | | 1.15 |
| Tij 2 20%fr. 2nd 10% | Ii | B-9 | | 1.18 | | **1.29** | | **6.89** |
| Ogunsheye 10% 2X | Ii | C-8 | | 1.16 | | **11.9** | | **10.83** |
| Hog | H | C-9 | | 1.17 | | 1.19 | | 1.22 |
| Beach φ insol. | B | C-10 | | 1.15 | | 1.26 | | 1.31 |
| J.S φ insol. | A | C-11 | | 1.17 | | 1.14 | | 1.16 |
| HCA | | D-7 | | 1.16 | | **8.8** | | **10.27** |
| HCA 1:5 Dil | | D-8 | | 1.16 | | **2.49** | | **2.87** |
| Tn-Artigen HAS | Tn | H-8 | | 1.14 | | 1.17 | | 1.17 |
| T-Antigen HAS | T | H-9 | | 1.19 | | 1.18 | | 1.15 |
| FITC-dextran | Ctrl-FITC | H-12 | | **58.66** | | **53.12** | | **39.2** |
| *Int/Bk., Ratio of mean flurescence intensity to mean background. | | | | | | | | |

### Antibody G1 Recognizes Triple Negative Breast Tumor Cell (including CSC-like cell) -associated differential expression of gp^{C1}

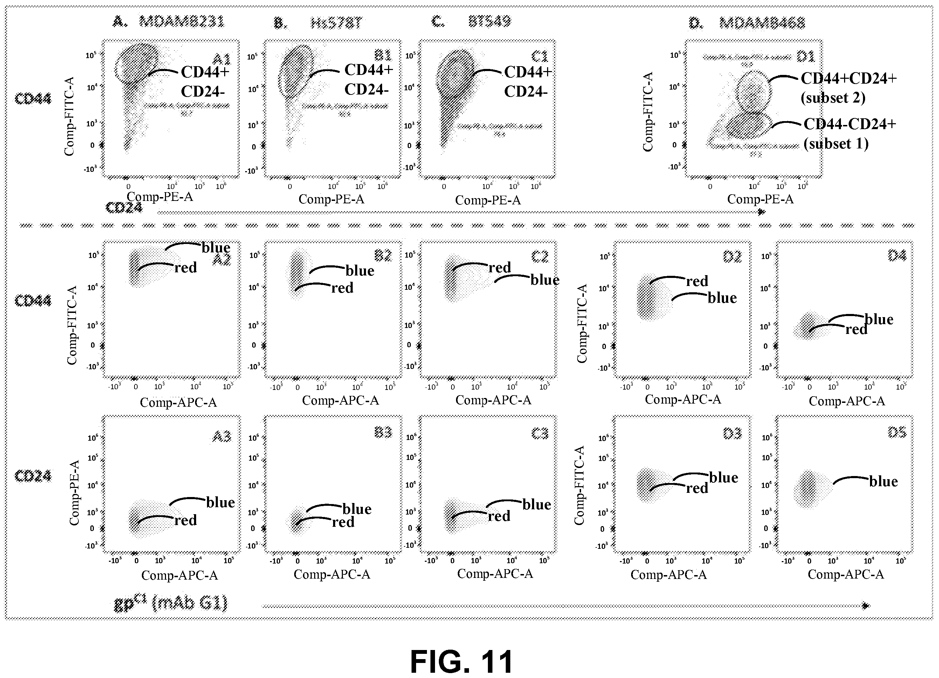

FIG. 11 illustrates example experimental results of staining cell lines using antibody G1 and CSC biomarkers, in accordance with various embodiments. In accordance with various experimental embodiments, antibody G1 is shown to recognize differential expression of gpC1 among triple negative breast tumor cell lines (TNBC), including CSCs. A panel of TNBC lines are FACS stained using mAb G1 and CSC markers CD44 and CD24. As illustrated by FIG. 11, two of the three CSC-like TNBC lines (CD44+CD24-), MDAMB-231 and BT549, are strongly gp^{C1+}.

More specifically, FIG. 11 illustrates that antibody G1 recognizes differential expression of gpC1 among TNBC lines. G1 or an isotype control mAb 9.14.7 was applied at 1.0µg per staining in combination with anti-CD44 and anti-CD24. The isotype control signal is in a first color (e.g., red) contour plot, G1-staining signal is in a second color (e.g., blue) contour plot. As illustrated by bottom two rows of FIG. 11 (labelled CD44 and CD24), MDAMB-231 and BT549 are stained strongly gpC1+, MDAMB-46 is intermediate positive, while Hs578T is weakly stained. As illustrated by the circled areas of the top row of FIG. 11, MDAMB-231 (A), Hs578T (B) and BT549 (C) are characteristically CSC cells with a CD44+CD24- main population. By contrast, also illustrated by the two circles areas in the top row of FIG. 11 related to MDAMB-468, MDAMB-468 is composed of two sub populations, CD44+CD24+ (D1, subset 2) and CD44-CD24+ (D1, subset 1).

It is noteworthy that more than 1 million global cases of BCa are diagnosed each year and approximately 15% are triple negative. Owing to the lack of an effective therapeutic target, a younger age at onset, and early metastatic spread, patients suffering triple-negative BCa often have poor prognoses and clinical outcomes. Use of gpC1 for diagnosis of and/or treatment of the triple-negative BCa can provide a variety of benefits. For example, the *O-*core cryptic glycan biomarker can be used for immunotype-enhanced precision diagnosis and prognosis of BCa and targeted immunotherapy against BCa metastasis.

Although tumor-associated abnormal glycosylation has been recognized for years, identifying glycan biomarkers of CSCs and CTCs remains technically challenging. Embodiments described herein include a practical approach to overcome this difficulty. Conceptually, the approach utilizes the fact that the immune systems of many animal species are able to recognize subtle changes in sugar moieties displayed by cells or soluble antigens and produce specific antibodies for abnormally expressed tumor glycan biomarkers. Experimentally, anti-tumor mAbs are first screened using carbohydrate microarrays to identify those that are specific for glycan biomarkers. Subsequently, it is determined whether the selected mAbs are specific for the cell-surface glycan biomarkers using flow cytometry and FAST-scan technology. Finally, the new antibody is used to probe to monitor CSC and/or CTC-expression of corresponding glycan biomarkers in advanced breast cancer patients. This approach is generally useful for exploring potential glycan biomarkers of CSCs and CTCs of epithelial cancers. The identified glycan biomarkers are demonstrated as successfully identifying CSCs and CTCs in blood samples, which can be used to monitor expression overtime, monitor treatment efficacy, and efficacy of drug candidates.

Although the embodiments illustrated by the various experimental embodiments describe identification of CSCs within a cell population of a blood sample of a human, embodiments are not so limited. For example, in various embodiments, CSCs can be detected from other organisms, such as various vertebrates and/or mammals including dogs, cats, horses, livestock, birds, fish, etc. The blood sample used is from the specific organism. Further, the antibodies used as targets are not limited to those identified herein and can include a variety of antibodies.

Terms to exemplify orientation, such as on top, onto, within, may be used herein to refer to relative positions of elements as shown in the figures. It should be understood that the terminology is used for notational convenience only and that in actual use the disclosed structures may be oriented different from the orientation shown in the figures. Thus, the terms should not be construed in a limiting manner.

As illustrated, various modules and/or other circuit-based building blocks (shown in the immediately preceding figure) may be implemented to carry out one or more of the operations and activities described herein, and/or shown in the block-diagram-type figures. In such contexts, these modules and/or building blocks represent circuits that carry out one or more of these or related operations/activities. For example, as discussed above, one or more modules and/or blocks are discrete logic circuits or programmable logic circuits configured for implementing these operations/activities, as in the circuit modules/blocks (e.g., the cell picking circuitry, processing circuitry, optical circuitry, and fluorescent microscope) shown aboveThe programmable circuit may be one or more computer circuits programmed to execute a set (or sets) of instructions (and/or configuration data). The instructions (and/or configuration data) can be in the form of firmware or software stored in and accessible from a memory (circuit). As an example, first and second modules/blocks include a combination of a CPU hardware-based circuit and a set of instructions in the form of firmware, where the first module/ block includes a first CPU hardware circuit with one set of instructions and the second module/block includes a second CPU hardware circuit with another set of instructions.

Various embodiments described above may be implemented together and/or in other manners. One or more of the items depicted in the present disclosure can also be implemented separately or in a more integrated manner, or removed and/or rendered as inoperable in certain cases, as is useful in accordance with particular applications. For example, the particular structures illustrated as shown and discussed may be replaced with other structures and/or combined together in the same apparatus. As another example, the methods illustrated by FIGs. 2 and 3 and can be implemented using the apparatus illustrated by FIG. 1. Further, the methods described by FIGs. 2-3 can be implemented together, separately, and/or using various combinations of the steps described therein. In view of the description herein, those skilled in the art will recognize that many changes may be made thereto without departing from the scope of the present disclosure.

## Claims

1. A method for detecting circulating cancer stem cells (CSCs) in a biological sample of a subject, the method comprising:
causing a physical interaction between the biological sample and an antibody by exposing the biological sample to the antibody; and
analyzing a cell population of the biological sample by:
determining a presence of CSCs within the cell population by detecting binding between the antibody and a glycan biomarker, the glycan biomarker including at least one chain selected from the group consisting of:
polylactosamine chains, oligosaccharide chains, and combinations thereof, the at least one chain having branches selected from the group consisting of: IIβ (Galβ1,4GlcNAcβ1,6), Iβ(Galβ1,3GlcNAcβ1,6), IIβ/Iβ (Gal β1, 4/3GlcNAc β1,6) -moieties, and combinations thereof; and
detecting a level of the CSCs within the cell population of the biological sample responsive to the detection of binding between the antibody and the glycan biomarker.

2. The method of claim 1, wherein analyzing the cell population further includes:
immunotyping the CSCs and circulating tumor cells (CTCs) within the cell population responsive to the detected binding between the antibody and the glycan biomarker.

3. The method of claim 1, further including:
classifying the cell population as CSCs, circulating tumor cells (CTCs), and benign cells responsive to the detected binding between the antibody and the glycan biomarker.

4. The method of claim 3, wherein cells of the cell population are classified as blood circulating CSCs (cCSCs), tissue-associated CSCs (tCSCs), and CTCs in blood circulation (cCTC).

5. The method of claim 1, wherein the glycan biomarker includes an epitope of a blood group precursor antigen and the antibody is an anti-tumor monoclonal antibody is C1, HAE3, or G1.

6. The method of claim 1, wherein determining the presence of the CSCs in the biological sample further includes using optical circuitry to detect the binding by identifying the specific binding of the glycan biomarker by the antibody within the biological sample.

7. The method of claim 1, wherein the glycan biomarker includes an *O*-core cryptic epitope of a blood group precursor antigen, the blood group precursor antigen selected from the group consisting of: Tij II 20% fraction 2nd 10% (Tij II), OG 10% 2X (OG), and a combination thereof.

8. The method of claim 1, wherein analyzing the cell population of the biological sample further includes immunotyping the CSCs and circulating tumor cells (CTCs) within the biological sample responsive to the detected binding between the antibody and the glycan biomarker and based on differences in morphology of the CSCs and CTCs.

9. The method of claim 1, wherein causing the physical interaction between the biological sample and the antibody further includes:
immobilizing the biological sample on a substrate and exposing the immobilized biological sample to the antibody and a detection agent, wherein the presence of the glycan biomarker within the biological sample results in the antibody binding to the glycan biomarker and binding of the detection agent to an FC segment of the antibody.

10. The method of claim 7, wherein:
determining the presence of the CSCs within the cell population further includes:
identifying the presence of the antibody bound to the glycan biomarker within the cell population, and
detecting the presence of the CSCs within the cell population of the biological sample responsive to the identified presence of the antibody bound to the glycan biomarker; and
analyzing the cell population of the biological sample includes characterizing at least a portion of the cell population as CSCs responsive to the identified presence of the antibody bound the glycan biomarker and using morphological and immunological analysis via a fiber-optic array scanning technology (FAST) scan to distinguish CSCs from benign cells in the cell population.

11. The method of claim 10, wherein analyzing the cell population of the biological sample further includes classifying at least a portion of the cell population as CSCs and at least another portion as circulating tumor cells, by morphological and immunological analysis.

12. The method of claim 10, wherein the biomarker includes a plurality of oligosaccharide chains having branches selected from the group consisting of: IIβ (Galβ1,4GlcNAcβ1,6), Iβ(Galβ1,3GlcNAcβ1,6), and combinations thereof, the method further including detecting a presence of metastatic cancer in the biological sample responsive to the detected level of the CSCs within the cell population.

13. The method of claim 1, wherein analyzing the cell population further includes comparing the detected level of the CSCs within the cell population to a previously determined level of CSCs of a different biological sample of the subject.

14. The method of claim 1, wherein:
the glycan biomarker includes an *O*-core cryptic epitope of a blood group precursor antigen, the blood group precursor antigen being selected from the group consisting of: Tij II 20% fraction 2nd 10% (Tij II), OG 10% 2X (OG), and a combination thereof; and
analyzing the cell population further includes identifying cancerous cells associated with an epithelial cancer in response to the detected level of the CSCs.

15. The method of claim 1, furthering including determining an efficacy of a drug candidate compound for treatment of cancer in a subject by:
obtaining a plurality of biological samples from blood or tissue of a subject before and after treatment with the drug candidate compound, the biological samples comprising a cell population suspected of containing CSCs, the plurality of biological samples including the biological sample;
causing physical interactions between each of the plurality of biological samples and the antibody by exposing the plurality of biological samples to the antibody; and
analyzing the cell population by identifying levels of the CSCs within the biological samples before treatment with the drug candidate compound compared to after treatment with the drug candidate compound, wherein the presence of a decreased number of the CSCs after treatment compared to a number of the CSCs before treatment indicates a relative efficacy of the drug candidate compound.

## Patentansprüche

1. Verfahren zum Nachweisen im Kreislauf befindlicher Krebs-Stammzellen (Cancer Stern Cells, CSCs) in einer biologischen Probe eines Individuums, wobei das Verfahren Folgendes umfasst:
Auslösen einer physikalischen Wechselwirkung zwischen der biologischen Probe und einem Antikörper, indem die biologische Probe mit dem Antikörper in Kontakt gebracht wird; und
Analysieren einer Zellpopulation der biologischen Probe, indem:
ein Vorliegen von CSCs in der Zellpopulation durch Nachweisen von Bindung zwischen dem Antikörper und einem Glycan-Biomarker bestimmt wird, wobei der Glycan-Biomarker wenigstens eine Kette enthält, die ausgewählt ist aus der Gruppe bestehend aus:
Polylactosaminketten, Oligosaccharidketten und Kombinationen davon,
wobei die wenigstens eine Kette Verzweigungen aufweist, die ausgewählt sind aus der Gruppe bestehend aus: IIβ (Galβ1,4GlcNAcβ1,6), Iß (Galβ1,3GlcNAcβ1,6), IIβ/Iβ (Galβ1,4/3GlcNAcβ1,6)-Gruppierungen und Kombinationen davon; und
ein Niveau der CSCs in der Zellpopulation der biologischen Probe, die beim Nachweis von Bindung zwischen dem Antikörper und dem Glycan-Biomarker eine Reaktion zeigt, nachgewiesen wird.

2. Verfahren nach Anspruch 1, wobei Analysieren der Zellpopulation ferner Folgendes umfasst:
Immuntypisieren der CSCs und im Kreislauf befindlicher Tumorzellen (Circulating Tumor Cells, CTCs) in der Zellpopulation, die bei der nachgewiesenen Bindung zwischen dem Antikörper und dem Glycan-Biomarker eine Reaktion zeigt.

3. Verfahren nach Anspruch 1, ferner umfassend:
Klassifizieren der Zellpopulation als CSCs, im Kreislauf befindliche Tumorzellen (CTCs) und gutartige Zellen, die bei der nachgewiesenen Bindung zwischen dem Antikörper und dem Glycan-Biomarker eine Reaktion zeigen.

4. Verfahren nach Anspruch 3, wobei Zellen der Zellpopulation als im Blutkreislauf befindliche CSCs (circulating CSCs, cCSCs), gewebeassoziierte CSCs (tissue-associated CSCs, tCSCs) und CTCs im Blutkreislauf (cCTC) klassifiziert werden.

5. Verfahren nach Anspruch 1, wobei der Glycan-Biomarker ein Epitop eines Blutgruppen-Vorläuferantigens umfasst und der Antikörper ein monoklonaler Antitumor-Antikörper ist, bei dem es sich um Cl, HAE3 oder G1 handelt.

6. Verfahren nach Anspruch 1, wobei Bestimmen des Vorliegens der CSCs in der biologischen Probe ferner Verwenden optischer Schaltkreise zum Nachweisen der Bindung durch Identifizieren der spezifischen Bindung des Glycan-Biomarkers durch den Antikörper in der biologischen Probe umfasst.

7. Verfahren nach Anspruch 1, wobei der Glycan-Biomarker ein kryptisches *O*-Kernepitop eines Blutgruppen-Vorläuferantigens umfasst, wobei das Blutgruppen-Vorläuferantigen ausgewählt ist aus der Gruppe bestehend aus: Tij II 20% Fraktion 2. 10% (Tij II), OG 10% 2X (OG) und einer Kombination davon.

8. Verfahren nach Anspruch 1, wobei Analysieren der Zellpopulation der biologischen Probe ferner Immuntypisieren der CSCs und im Kreislauf befindlicher Tumorzellen (CTCs) in der biologischen Probe, die bei der nachgewiesenen Bindung zwischen dem Antikörper und dem Glycan-Biomarker eine Reaktion zeigen, und bezogen auf Unterschiede in der Morphologie der CSCs und CTCs umfasst.

9. Verfahren nach Anspruch 1, wobei Auslösen der physikalischen Wechselwirkung zwischen der biologischen Probe und dem Antikörper ferner Folgendes umfasst:
Immobilisieren der biologischen Probe an einem Substrat und In-Kontakt-Bringen der immobilisierten biologischen Probe mit dem Antikörper und einem Nachweismittel, wobei das Vorliegen des Glycan-Biomarkers in der biologischen Probe zur Bindung des Antikörpers an den Glycan-Biomarker sowie Bindung des Nachweismittels an ein FC-Segment des Antikörpers führt.

10. Verfahren nach Anspruch 7, wobei:
Bestimmen des Vorliegens der CSCs in der Zellpopulation ferner Folgendes umfasst:
Identifizieren des Vorliegens des an den Glycan-Biomarker in der Zellpopulation gebundenen Antikörpers und
Nachweisen des Vorliegens der CSCs in der Zellpopulation der biologischen Probe, die bei dem identifizierten Vorliegen des an den Glycan-Biomarker gebundenen Antikörpers eine Reaktion zeigt; und
Analysieren der Zellpopulation der biologischen Probe Charakterisieren wenigstens eines Teils der Zellpopulation als CSCs, die beim identifizierten Vorliegen des an den Glycan-Biomarker gebundenen Antikörpers eine Reaktion zeigen, und Anwenden einer morphologischen und immunologischen Analyse über einen FAST (Fiber-optic Array Scanning Technology)-Scan, um CSCs von gutartigen Zellen in der Zellpopulation zu unterscheiden, umfasst.

11. Verfahren nach Anspruch 10, wobei Analysieren der Zellpopulation der biologischen Probe ferner Klassifizieren wenigstens eines Teils der Zellpopulation als CSCs und wenigstens eines anderen Teils als im Kreislauf befindliche Tumorzellen anhand morphologischer und immunologischen Analyse umfasst.

12. Verfahren nach Anspruch 10, wobei der Biomarker mehrere Oligosaccharidketten umfasst, die Verzweigungen aufweisen, die ausgewählt sind aus der Gruppe bestehend aus: IIβ (Galβ1,4GlcNAcβ1,6), Iß (Galβ1,3GlcNAcβ1,6) und Kombinationen davon, wobei das Verfahren ferner Nachweisen eines Vorliegens einer metastatischen Krebserkrankung in der biologischen Probe, die bei dem nachgewiesenen Niveau der CSCs in der Zellpopulation eine Reaktion zeigt, umfasst.

13. Verfahren nach Anspruch 1, wobei Analysieren der Zellpopulation ferner Vergleichen des nachgewiesenen Niveaus der CSCs in der Zellpopulation mit einem zuvor bestimmten Niveau von CSCs einer anderen biologischen Probe des Individuums umfasst.

14. Verfahren nach Anspruch 1, wobei:
wobei der Glycan-Biomarker ein kryptisches *O-*Kernepitop eines Blutgruppen-Vorläuferantigens umfasst, wobei das Blutgruppen-Vorläuferantigen ausgewählt ist aus der Gruppe bestehend aus: Tij II 20% Fraktion 2. 10% (Tij II), OG 10% 2X (OG) und einer Kombination davon; und
Analysieren der Zellpopulation ferner Identifizieren mit einem Epithelkarzinom assoziierter Krebszellen als Reaktion auf das nachgewiesene Niveau der CSCs umfasst.

15. Verfahren nach Anspruch 1, weiterhin umfassend Bestimmen einer Wirksamkeit einer Kandidatenarzneistoffverbindung zur Behandlung von Krebs bei einem Individuum durch:
Erhalten mehrerer biologischer Proben aus Blut oder Gewebe eines Individuums vor und nach Behandlung mit der Kandidatenarzneistoffverbindung, wobei die biologischen Proben eine Zellpopulation umfassen, bei der der Verdacht besteht, dass sie CSCs enthält, wobei die mehreren biologischen Proben die biologische Probe enthalten;
Auslösen physikalischer Wechselwirkungen zwischen jeder der mehreren biologischen Proben und dem Antikörper, indem die mehreren biologischen Proben mit dem Antikörper in Kontakt gebracht werden; und
Analysieren der Zellpopulation durch Identifizieren von Niveaus der CSCs in den biologischen Proben vor Behandlung mit der Kandidatenarzneistoffverbindung gegenüber nach Behandlung mit der Kandidatenarzneistoffverbindung, wobei das Vorliegen einer verminderten Anzahl der CSCs nach Behandlung gegenüber einer Anzahl der CSCs vor Behandlung eine relative Wirksamkeit der Kandidatenarzneistoffverbindung anzeigt.

## Revendications

1. Procédé pour la détection de cellules souches cancéreuses (CSC) circulantes dans un échantillon biologique d'un sujet, le procédé comprenant :
le fait de provoquer une interaction physique entre l'échantillon biologique et un anticorps en exposant l'échantillon biologique à l'anticorps ; et
l'analyse d'une population de cellules de l'échantillon biologique par :
la détermination d'une présence de CSC dans la population de cellules en détectant une liaison entre l'anticorps et un biomarqueur de type glycane, le biomarqueur de type glycane comprenant au moins une chaîne choisie dans le groupe constitué par :
des chaînes de polylactosamine, des chaînes d'oligosaccharides, et des combinaisons correspondantes, l'au moins une chaîne possédant des ramifications choisies dans le groupe constitué par : des groupements IIβ (Galβ1,4GlcNAcβ1,6), Iβ(Galβ 1,3GlcNAcβ1,6), IIβ/Iβ (Galβ1, 4/3GlcNAcβ1,6), et des combinaisons correspondantes ; et
la détection d'un taux de CSC, dans la population de cellules de l'échantillon biologique sensibles à la détection d'une liaison entre l'anticorps et le biomarqueur de type glycane.

2. Procédé selon la revendication 1, l'analyse de la population cellulaire comprenant en outre :
l'immunotypage des CSC et de cellules tumorales circulantes (CTC), dans la population de cellules sensibles à la liaison détectée entre l'anticorps et le biomarqueur de type glycane.

3. Procédé selon la revendication 1, comprenant en outre :
la classification de la population de cellules comme CSC, cellules tumorales circulantes (CTC), et cellules bénignes sensibles à la liaison détectée entre l'anticorps et le biomarqueur de type glycane.

4. Procédé selon la revendication 3, des cellules de la population de cellules étant classifiées comme CSC circulantes dans le sang (cCSC), CSC associées à un tissu (tCSC), et CTC dans la circulation de sang (cCTC).

5. Procédé selon la revendication 1, le biomarqueur de type glycane comprenant un épitope d'un antigène précurseur de groupe sanguin et l'anticorps étant un anticorps monoclonal antitumoral qui est C1, HAE3 ou G1.

6. Procédé selon la revendication 1, la détermination de la présence des CSC dans l'échantillon biologique comprenant en outre l'utilisation d'une circuiterie optique pour détecter la liaison en identifiant la liaison spécifique du biomarqueur de type glycane par l'anticorps dans l'échantillon biologique.

7. Procédé selon la revendication 1, le biomarqueur de type glycane comprenant un épitope cryptique à noyau *O* d'un antigène précurseur de groupe sanguin choisi dans le groupe constitué par Tij II 20 % fraction 2^{d} 10 % (Tij II), OG 10 % 2X (OG), et une combinaison correspondante.

8. Procédé selon la revendication 1, l'analyse de la population de cellules de l'échantillon biologique comprenant en outre un immunotypage des CSC et des cellules tumorales circulantes (CTC) dans l'échantillon biologique sensibles à la liaison détectée entre l'anticorps et le biomarqueur de type glycane et sur la base de différences de morphologie des CSC et des CTC.

9. Procédé selon la revendication 1, le fait de provoquer l'interaction physique entre l'échantillon biologique et l'anticorps comprenant en outre :
l'immobilisation de l'échantillon biologique sur un substrat et l'exposition de l'échantillon biologique immobilisé à l'anticorps et à un agent de détection, la présence du biomarqueur de type glycane dans l'échantillon biologique entraînant la liaison de l'anticorps au biomarqueur de type glycane et la liaison de l'agent de détection à un segment FC de l'anticorps.

10. Procédé selon la revendication 7,
la détermination de la présence des CSC dans la population de cellules comprenant en outre :
l'identification de la présence de l'anticorps lié au biomarqueur de type glycane dans la population de cellules, et
la détection de la présence des CSC dans la population de cellules de l'échantillon biologique sensibles à la présence identifiée de l'anticorps lié au biomarqueur de type glycane ; et
l'analyse de la population de cellules de l'échantillon biologique comprenant la caractérisation d'au moins une partie de la population de cellules comme CSC sensibles à la présence identifiée de l'anticorps lié au biomarqueur de type glycane et l'utilisation d'une analyse morphologique et immunologique via un balayage par une technologie de balayage de réseaux par fibre optique (FAST) pour distinguer des CSC de cellules bénignes dans la population de cellules.

11. Procédé selon la revendication 10, l'analyse de la population de cellules de l'échantillon biologique comprenant en outre la classification d'au moins une partie de la population de cellules comme CSC et au moins une autre partie comme cellules tumorales circulantes, par une analyse morphologique et immunologique.

12. Procédé selon la revendication 10, le biomarqueur comprenant une pluralité de chaînes d'oligosaccharides possédant des ramifications choisies dans le groupe constitué par : IIβ (Galβ1,4GlcNAcβ1,6), Iβ(Galβ1,3GlcNAc β1,6), et des combinaisons correspondantes, le procédé comprenant en outre la détection d'une présence d'un cancer métastatique dans l'échantillon biologique sensible au taux détecté des CSC dans la population de cellules.

13. Procédé selon la revendication 1, l'analyse de la population de cellules comprenant en outre la comparaison du taux détecté des CSC dans la population de cellules avec un taux précédemment déterminé de CSC d'un échantillon biologique différent du sujet.

14. Procédé selon la revendication 1,
le biomarqueur de type glycane comprenant un épitope cryptique à noyau *O* d'un antigène précurseur de groupe sanguin, l'antigène précurseur de groupe sanguin étant choisi dans le groupe constitué par Tij II 20 % fraction 2^{d} 10 % (Tij II), OG 10 % 2X (OG), et une combinaison correspondante ; et l'analyse de la population de cellules comprenant en outre l'identification de cellules cancéreuses associées à un cancer épithélial en réponse au taux détecté des CSC.

15. Procédé selon la revendication 1, comprenant en outre la détermination d'une efficacité d'un composé médicamenteux candidat pour le traitement d'un cancer chez un sujet par :
l'obtention d'une pluralité d'échantillons biologiques à partir de sang ou de tissu d'un sujet avant et après traitement avec le composé médicamenteux candidat, les échantillons biologiques comprenant une population de cellules dont on suspecte qu'elles contiennent des CSC, la pluralité d'échantillons biologiques comprenant l'échantillon biologique ;
le fait de provoquer des interactions physiques entre chacun de la pluralité d'échantillons biologiques et l'anticorps en exposant la pluralité d'échantillons biologiques à l'anticorps ; et
l'analyse de la population de cellules en identifiant des taux de CSC dans l'échantillon biologique avant le traitement par le composé médicamenteux candidat par comparaison avec après le traitement par le composé médicamenteux candidat, la présence d'un nombre diminué de CSC après traitement par comparaison avec un nombre de CSC avant traitement indiquant une efficacité relative du composé médicamenteux candidat.
